(19) Europäisches Patentamt
European Patent Office
Office européen des brevets

(11) **EP 1 581 896 B1**

(12) **EUROPÄISCHE PATENTSCHRIFT**

(45) Veröffentlichungstag und Bekanntmachung des Hinweises auf die Patenterteilung:
**13.07.2016 Patentblatt 2016/28**

(21) Anmeldenummer: **03785641.6**

(22) Anmeldetag: **10.11.2003**

(51) Int Cl.:
*G06F 17/50* (2006.01)    *G05B 19/4097* (2006.01)
*G06F 3/00* (2006.01)    *G06T 9/00* (2006.01)
*A61C 13/00* (2006.01)

(86) Internationale Anmeldenummer:
**PCT/EP2003/012525**

(87) Internationale Veröffentlichungsnummer:
**WO 2004/044787 (27.05.2004 Gazette 2004/22)**

(54) **VERFAHREN ZUR HERSTELLUNG VON ZAHNERSATZTEILEN ODER ZAHNRESTAURATIONEN UNTER VERWENDUNG ELEKTRONISCHER ZAHNDARSTELLUNGEN**

METHOD FOR PRODUCING DENTURE PARTS OR TOOTH RESTORATIONS USING ELECTRONIC DENTAL REPRESENTATIONS

PROCÉDÉ DE PRODUCTION DE PIÈCES PROTHÉTIQUES DENTAIRES OU DE RESTAURATIONS DENTAIRES AU MOYEN DE REPRÉSENTATIONS DENTAIRES ÉLECTRONIQUES

(84) Benannte Vertragsstaaten:
**AT CH DE DK ES FR GB IT LI SE**

(30) Priorität: **11.11.2002 DE 10252298**

(43) Veröffentlichungstag der Anmeldung:
**05.10.2005 Patentblatt 2005/40**

(60) Teilanmeldung:
**16152909.4**

(73) Patentinhaber: **Sirona Dental Systems GmbH
64625 Bensheim (DE)**

(72) Erfinder: **MEHL, Albert
8125 Zollikerberg (CH)**

(74) Vertreter: **Sommer, Peter et al
PRIO Patentanwälte
Augustaanlage 22
68165 Mannheim (DE)**

(56) Entgegenhaltungen:
EP-A- 0 667 587    EP-A- 1 276 072
EP-A- 1 304 088    WO-A-99/59106
US-A- 5 092 022    US-A- 5 257 203
US-A1- 2002 015 934

- SALIGER, G: "CAD/CAM in aesthetic dentistry; Designing a CEREC crown" QUENTESSENCE, CHICAGO, 1996 ED. W:H: MÖRMANN, XP001180141
- R. LUTHARDT P. KÜHMSTEDT P. BRAKHAGE: "Digitalisierung vollständiger Kiefermodelle und CAD-Modellation von Okklusalflächen" NEUE TECHNOLOGIE, XP001180082
- A. MEHL, W. GLOGER, R. HICKEL: "Erzeugung von CAD-Datensätzen für Inlays und Kronen mit funktionellen Kauflächen" ORIGINALARBEIT, XP001180080
- BLANZ, V. ROMDHANI, S: "Face Identification across different Poses and illuminations with a 3D Morphable Model" 2002 IEEE, XP001180087

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Herstellung eines allgemeinen dreidimensionalen elektronischen Bildes eines Zahns und dessen Verwendung zur Herstellung von Zahnmodellen, Zahnersatzteilen oder Zahnrestaurationen reparaturbedürftiger Zähne bzw. Defektsituationen.

Für die Versorgung von Zahndefekten stehen verschiedene Möglichkeiten zur Verfügung. Eine Möglichkeit ist die direkte Applikation von Füllungsmaterial im Mund, d.h. der Zahnarzt entfernt die Karies und füllt das Loch in der gleichen Sitzung mit einem Füllungsmaterial. Diese Vorgehensweise wird vor allem bei kleineren Defekten gewählt. Für größere Defekte greift man eher auf Materialien wie Metall oder Keramiken etc. zurück, die nicht direkt im Mund angefertigt werden können. Auch ist die Kauflächengestaltung im Mund bei größeren Defekten eher problematisch und schwer durchzuführen. Daher wird beim Zahnarzt nach erfolgter Präparation des Zahnes eine Abformung durchgeführt. Diese Abformung wird in ein zahntechnisches Labor gegeben und ein Gipsmodell erstellt. Unter Berücksichtigung der Gegenbezahnung und eventuell unter Einbeziehung der Kieferbewegungen in Form von Artikulatoren kann dann die entsprechende Zahnrestauration oder Zahnersatzteil angefertigt werden. Dies können z.B. Inlays, Onlays, Teilkronen, Kronen, Brücken, Teleskopkronen, Teilprothesen etc. sein. Die Herstellung einer solchen Restauration ist allerdings sehr aufwendig. Nach der Abformung und Gipsmodellherstellung mit Zuordnung des Gegenkiefers erfolgt das Aufwachsen bzw. Sintern, Einbetten, Gießen oder Pressen, Ausarbeiten, Aufpassen und Polieren. Die hohe Anzahl der Schritte und die beschränkten technischen Möglichkeiten im zahntechnischen Labor führen dazu, dass zum einen sich Verarbeitungsfehler einschleichen können und die Materialqualität der fertigen Versorgung nicht optimal ist und zum anderen nicht alle Materialien gefertigt werden können (z.B. Hochleistungskeramiken). Zusätzlich führt der hohe Arbeitsaufwand auch zu hohen Kosten.

Als Alternative zur konventionellen Herstellungsweise wird inzwischen die CAD/CAM-Technologie gesehen, bei der mit Unterstützung von Computerverfahren die Anfertigung

von Zahnrestaurationen und Zahnersatzteile durchgeführt wird. Vereinfacht gesprochen gliedert sich der Prozess in:

1. dreidimensionaler Datenerfassung der Präparation bzw. mehrere Präparationen

2. Erzeugung eines CAD-Datensatzes der Zahnestauration, d.h. Konstruktion bzw. Berechnung der Außenhülle und/oder interaktive Modellation der Außenhülle am Bildschirm

3. Bearbeitung des fertigen CAD-Datensatzes in einer computergesteuerten Fräs- bzw. Schleifinaschine (z.B. CNC) oder Rapid-Prototyping.Systemen.

Der Vorteil eines solchen Verfahrens liegt auf der Hand:

1. Kostenersparnis durch Automatisierung und damit Zeitersparnis

2. Verwendung industriell gefertigter Materialien. Diese können unter optimaleren Bedingungen als im Labor gesintert, gegossen etc. werden und weisen daher bessere Materialeigenschaften auf. Speziell für Keramiken und Titan sind diese Vorteile bereits eingehend untersucht worden.

3. Zahnersatz wird mit gleichbleibender Qualität produziert. Es kommt zu keinen Schwankungen durch Verarbeitungsfehler wie bei konventionellen Herstellungsprozessen.

4. Völlig neuartige Materialien wie Zirkonoxid-Keramiken etc, die bisher im konventionellen zahntechnischen Prozess nicht oder nur mit hohem Aufwand zu bearbeiten waren, lassen sich mit dem CNC-Verfahren anfertigen.

Einige Systeme sind bereits im Einsatz. Eine aktuelle Übersicht ist zum Beispiel der Ausgabe der ZWP (Dez. 2001, Mehl) zu entnehmen. Des weiteren werden in den Patentschriften US 5,217,375, EP 0 643 948, EP 0 634 150, EP 0 913 130 A2 und WO 0239056 solche Systeme bzw. einzelne Aspekte solcher Systeme beschrieben.

Ein Problem, das bisher nicht gelöst wurde, ist die möglichst automatisierte Herstellung von Zahnrestaurationen, die bereits eine Kaufläche aufweisen, die allen funktionellen und morphologischen Kriterien einer Kaufläche entspricht und optimal an die Gegenzahnsituation angepasst werden kann.

Bei den meisten Systemen ist zur Zeit nur die Herstellung von Gerüsten möglich. Ähnlich der konventionellen Vorgehensweise, bei der man z.B. ein Metallgerüst mit Keramik oder Kunststoff verblendet (gilt auch für andere Materialien wie spezielle Keramik- oder Kunststoffgerüste), wird das Grundgerüst im CAD/CAM-Prozess erstellt und anschließend zumindest Teile der Kaufläche und noch fehlende Außenflächen konventionell mit Keramik, Komposit etc. verblendet. Diese Gerüste können z.B. in der CAD-Software (Konstruktionssoftware) durch Vergrößerung der Präparation bzw. Berechnung einer Oberfläche, die in einem bestimmten, wählbaren Abstand (=Schichtstärke des Gerüstes) zur Präparationsoberfläche liegt, erreicht werden. Zusätzlich ist noch die Einbeziehung von "Ausbuchtungen" und "Deformationen" möglich. In EP 0 913 130 A2 ist mit Fig.13b solch ein Vorgehen zu sehen. Ein weiteres Beispiel ist in EP 0 643 948 A1 beschrieben.

Für die automatische Generierung einer Kaufläche, die nach allen gewünschten Kriterien und Anforderungen an eine gute Zahnrestauration bzw. Zahnersatzteil gestaltet ist, ist noch kein Verfahren vorhanden. Dies wäre jedoch von besonderer Bedeutung, da damit die Einsatzfähigkeit und die Kosteneffizienz eines CAD/CAM-Systems gesteigert und damit vor allem die CAD/CAM-Technologie überhaupt im großen Rahmen in der Zahnmedizin etabliert werden kann. Gleichzeitig muss mit diesem Verfahren auch die Möglichkeit bestehen, den berechneten Zahnersatz in einer Maschine anfertigen zu können.

Verschiedene Verfahren zur Kauflächengestaltung werden in der Literatur und in den Patentschriften beschrieben. Für die Rekonstruktion von Inlay-Flächen werden sowohl die Linearmethode als auch verschiedene Extrapolationsmethoden beschrieben (Mattiola, A., Mörmann, W.H. und Lutz, F.: "Computerunterstützte Okklusion von Cerec 2 Inlays und Overlays". Schweiz Monatsschr Zahnmed 105:1283-1290 (1995); Kunzelmann, K.-H., Mehl, A., Pelka, M.: Automatische Rekonstruktion von Kauflächen computergenerierter Restaurationen. Zahnärztl Welt/Rundschau 102, 695 - 703 (1993)). Bei der Linearmethode werden gegenüberliegende Punkte der Kavitätengrenze (meistens in oro-vestibulärer Richtung) einfach durch eine Gerade verbunden und so der Defekt aufgefüllt. Bei der Extrapolation wird die Steigung (Gradient) der noch vorhandenen Restzahnsubstanz in den Defekt hinein fortgesetzt und so die Oberfläche rekonstruiert. Es ist offensichtlich, das mit dieser Vorgehensweise nur angenähert etwas kauflächenähnliches entstehen kann. Die Einbeziehung morphologischer Kriterien und die Gegenbezahnungssituation ist nicht möglich. Gleichzeitig ist dieses Verfahren nur für relativ kleine Defekte geeignet.

Eine zweite Möglichkeit besteht in einer weiteren dreidimensionalen optischen Vermessung entweder der vorhandenen Kaufläche, bevor der Zahn beschliffen wird, oder einer individuell mit Wachs oder Kunststoff modellierten Kaufläche (z.B. Mattiola, A., Mörmann, W.H. und Lutz, F.: "Computerunterstützte Okklusion von Cerec 2 Inlays und Overlays". Schweiz Monatsschr Zahnmed 105:1283-1290 (1995), Mehl, A., Gloger, W., Hickel, R.: Erzeugung von CAD-Datensätzen für Inlays und Kronen mit funktionellen Kauflächen. Dtsch Zahnärztl Z 52, 520-524 (1997)). Durch Anklicken oder Auswahl von Referenzpunkten an den Nachbarzähnen kann die Präparations- und die Kauflächenmessung zueinander positioniert und die vollständige Restauration ergänzt werden. Hier muss allerdings eine Wachsmodellation manuell erstellt werden, so dass der Vorteil der Automatisierung durch das CAD/CAM-System nicht mehr gegeben ist. In den meisten Fällen bei der Versorgung eines Zahnes wird die Ausgangskaufläche aufgrund vorhandener kariöser Defekte oder insuffizienter Versorgungen nicht verwendbar sein, so dass auch diese Möglichkeit nur für einen kleinen begrenzten Indikationsbereich beschränkt bleibt. Eine zusätzliche Möglichkeit wird in WO 0239056 vorgestellt. Hier wird ein Patientenarchivierungssystem z.B. Chipkarte für den Patienten, beschrieben, das beinhaltet, das Zahndaten gespeichert werden. Diese Zahndaten können dann zu einem späteren Zeitpunkt, wenn beim Patienten Versorgungen angefertigt werden, wieder verwendet werden und für die Rekonstruktion des Defektes dienen. Hier wäre jedenfalls gewährleistet, dass die ergänzte Kaufläche morphologisch und funktionell optimal dem gnathologischen System angepasst ist. Allerdings muss man bei diesen Verfahren mit entsprechenden Vorlaufszeiten rechnen, so dass vorerst für die Versorgungen einer breiten Masse andere Verfahren herangezogen werden müssen.

Weitere Möglichkeiten im Zusammenhang mit der Einbeziehung von Kauflächengeometrien in den CAD/CAM-Prozess werden in den folgenden Erfindungen beschrieben. Aus DE 198 38 239 A1 sind Gruppen von Rohlingen für Dentalrestaurationen bekannt, die unterschiedlichen Zahntypen zugeordnet werden und deren Außengeometrien aus Mittelwerten, die einschlägigen Lehrbüchern entnommen

werden, für den jeweiligen Zahntyp bestimmt werden. Dabei handelt es sich aber nicht um eine mathematische, für die CAD/CAM-Rekonstruktion von Zahnrestaurationen verwendbare Beschreibung von Zahnoberflächen, sondern nur um eine ungefähre Maximum -Minimum Abschätzung für die groben Außenmaße von Rohlingen, aus denen dann die gewünschte individuelle Zahnrestauration herausgefräst werden soll. Die Mittelwerte, die aus der Literatur übernommen werden können, sind außerdem nur Längen-, Breiten- oder ähnliche lineare Messungen, die in keiner Weise auch nur annähernd eine Kaufläche für den computergestützten Rekonstruktionsprozess beschreiben können.

Aus DE 199 23 978 A1 ist ein Verfahren zur computergestützten patientenspezifischen Darstellung und Planung zahnärztlicher und/oder zahnprothetischer Arbeiten bekannt, bei denen eine digitalisierte Bilddatenbank mit einer Vielzahl von Modellzahn- und Kieferansichten erstellt wird, wobei die Modellansichten gesunde und dentale Krankheitsbefunde aufweisende Objekte, z.B. Einzelzähne umfassen, wobei die Bilddatenbank Abbildungen typischer Mundregionen beinhaltet. Dieses Verfahren dient als computergestütztes Expertensystem für die Therapieplanung und -entscheidung von zahnärztlichen Behandlungen. Für die dreidimensionale Rekonstruktion von Zahndefekten, wie es im CAD/CAM-Prozess zur Herstellung von Zahnersatz notwendig ist, ist dieses Verfahren nicht geeignet, da patientenspezifische Befunddaten nicht für die exakte individuelle Anpassung der Bilddatenbanken ausreichen. Die Bilddatenbank soll auch nur typische Standardformen für das Planungsgespräch mit dem Patienten zur Verfügung stellen, eine Veränderung durch Kombinationen dieser Daten zu einem neuen repräsentativen Datensatz wird nicht vorgenommen.

Aus EP 0 643 948 A1 ist ein Verfahren zur Herstellung einer Dentalrestauration bekannt, bei dem eine selbstlernende Datenbibliothek von Zahnbasisformen zum Einsatz kommt. Die Vorgehensweise beschränkt sich dabei nur auf die Erstellung von Kronengerüsten und beinhaltet das Lernen nur solcher Parameter wie Schichtdicke, Lokalisierung und Dicke von Wölbungen, ungefährer Verlauf der Präparationslinie. Dieses einfache "Lernen" führt nicht zu mathematischen oder parametrischen Beschreibungen von Zahnoberflächen, die für die Rekonstruktion von individuellen Zahndefekten mit kompletter Außengeometrie wie anatomisch und funktionell geformte Inlays, Onlays, Kronen und Brücken geeignet sind. Insbesondere kann hiermit auch keine Berücksichtigung der umgebenden Restgebisssituation wie Nachbarzähne und Gegenbezahnung stattfinden. Weiterhin wird hier nicht die von der Natur vorgegebene Form nachgebildet, sondern nur die auf der Erfahrung des/der Zahntechniker / Experten beruhenden physikalischen Konstruktionsparameter von Gerüsten ermittelt.

Aus US 5,257,203 ist ein Verfahren zur Herstellung einer Dentalrestauration bekannt, bei dem eine Datenbank standardisierter generischer Zahnformen eingesetzt wird, wobei diese generischen Zahnformen typischerweise computerbasierte Darstellungen von standardisierten Gipsmodellen von Zähnen sind. Das generisch Zahnmodell wird mittels mathematischer Methoden an die Präparationsstelle angepasst.

Bei den in diesem Verfahren benutzten generischen Zahnformen handelt es sich nicht um rechnerisch oder algorithmisch aus einer Datenbank abgeleitete Zahnformen und damit nicht um im Sinne der vorliegenden Erfindung beschriebene generische Zahnmodelle, sondern um standardisierte Gipsmodelle, die nur dreidimensional gescannt werden und dieser Datensatz für die Rekonstruktion verwendet wird. Der Nachteil ist auch hier, dass der Standardisierung kein allgemeingültiges Konstruktionsprinzip zu Grunde liegt und die Gestaltung nur auf der manuellen Geschicklichkeit und der Erfahrung einzelner Experten mit entsprechender Einschränkung der in der Natur vorkommenden Formvielfalt beruht.

Eine weitere Möglichkeit zur Herstellung von Zahnrestaurationen wird in "Saliger, G., Designing a CEREC crown. In Cerec 10 year anniversary Smposium, ed. W.H. Mörmann. Quintessence, Chicago, 1996" oder DE 19642247 beschrieben. Hier wird der Datensatz eines Modellzahnes an den präparierten Zahn angepasst und adaptiert. Im wesentlichen wird dieser Modellzahn entsprechend der mesial-distalen Ausdehnung des Defektes skaliert, translatiert und rotiert. Eine elastische Deformation kann das Ergebnis noch verbessern. Bei Saliger 1996 (s.o) wird eine anschließende interaktive Möglichkeit der Rotation und der Kontrolle der Kaufläche zum Gegenzahn bereitgestellt. Zusätzlich können die Höcker in ihrer Position verändert werden. Dies erfolgt alles interaktiv. Zum Schluss wird dann die Zahnrestauration gefräst.

Das Problem bei all den erwähnten Vorgehensweisen liegt vor allem in folgenden Fakten begründet:

- Kontaktpunkte zum Gegenzahn werden erst im nachhinein festgelegt, indem durch interaktive Verzerrungen die Anpassung erfolgt oder der Modellzahn solange verändert wird, bis Berührungen mit der Hülle vorliegen. Dies führt oft zu völlig zahnuntypischen Formen, da der Modellzahn von Anfang an nicht optimal in die Gesamtsituation angepasst wird.
- Es gibt keine automatisiertes Vorgehen für die Auswahl eines besten Modellzahnes (falls mehrere zur Auswahl stehen). Bis jetzt erfolgt das nur auf Grund visueller Regeln.
- Die Arbeit und interaktive Veränderung am Monitor ist in ihrer Auswirkung im dreidimensionalen nur schwer vorstellbar und daher für einen mit der Computer-Arbeit weniger Erfahrenen nur nach langer Einübungszeit und täglicher Routine zu beherrschen.
- Die Morphologie der Nachbarzähne bzw. Antagonisten oder auch der entsprechende im gleichen Kiefer gegenüberliegende Zahntyp wird nicht herangezogen. Dies ist in manchen Fällen wichtig, um eine harmonische Eingliederung der Restauration in das Kiefersystem zu gewährleisten.
- Veränderungen des Modellzahnes durch Skalierung, Höckerpositionierung und interaktiven Deformationen müssen nicht unbedingt zu zahnähnlichen Oberflächen führen.
- Für alle interaktiven oder automatischen Anpassungen gibt es bis jetzt kein Verfahren, dass garantiert, dass nach Veränderung wieder eine einem natürlichen Zahn sehr ähnliche Kaufläche entsteht. Nachdem bis heute die Kriterien einer guten funktionell und statisch gestalteten Kaufläche wissenschaftlich noch nicht bekannt und auch nachgewiesen sind, muss die Forderung für jede Restauration lauten, dass sie möglichst nahe an natürliche Gegebenheiten und Formen angenähert wird, um so keine Langzeitschäden an den Zähnen, im Gewebe und im Gelenk zu verursachen.

Eine Überwindung vorausgehend angesprochener Probleme ist mit der vorliegenden Erfindung gelungen, welche die Herstellung von Zahnrestaurationen, Zahnersatzteilen oder Zahnmodellen mit Kauflächen und/oder Oberflächen, die einem natürlichen Zahn sehr nahe kommen und sich funktionell und morphologisch optimal in die Kiefersituation einfügen, ermöglicht, wobei der Herstellungsvorgang mehr automatisiert werden kann, d.h. mit wesentlich weniger Interaktionen und fehlerfreier, d.h. benutzerfreundlicher, ablaufen kann.

Unter Zahnersatzteile versteht man in dieser Patentschrift Teile oder die Gesamtheit von Total- oder Teilprothesen (z.B. Teleskopprothese, Klammerprothese, Interimsprothesen etc.) oder auch Implantataufbauten, unter Zahnrestaurationen Brücken, Teleskopkronen (Primär- und Sekundärteile), Kronen, Inlays, Onlays, Overlays und Teilkronen. Zahnmodelle werden verwendet als Prothesenzähne, als eigenständige Modelle, als Bestandteile von Übungs-, Ausbildungs- und Anschauungsmodellen oder zur Darstellung in elektronischen Medien oder Printmedien. Abzugrenzen ist davon der Begriff des generischen Zahnmodells oder generischen Zahnmodelldatensatzes, wie er im Folgenden erklärt wird.

Die Erfindung schafft ein Verfahren zur Herstellung eines elektronischen Datensatzes eines für die Anfertigung eines Zahnersatzteils, einer Zahnrestauration oder eines Zahnmodells verwendbaren generischen Zahnmodells, wie es in Anspruch 1 angegeben ist. Dieses kann zur Herstellung von Zahnmodellen, Zahnersatzteilen oder Zahnrestaurationen verwendet werden. Eine Verwendung des Verfahrens zur Herstellung eines elektronischen Datensatzes des generischen Zahnmodells ist in Anspruch 4 angegeben. Eine Verwendung einer numerisch gesteuerten Maschine zur Herstellung von Zahnmodellen, Zahnersatzteilen oder Zahnrestaurationen, indem die Maschine entsprechend mit einem erfindungsgemäß erhaltenen Datensatz gesteuert wird, ist in Anspruch 14 angegeben. Weiterbildungen der erfindungsgemäßen Verfahren sind in den abhängigen Ansprüchen angegeben. In Anspruch 15 und 16 wird eine Vorrichtung zur Visualisierung, Anpassung und Justierung eines generischen Zahnmodelldatensatzes angegeben.

Ein auf erfindungsgemäße Weise erhaltener Datensatz eines generischen Zahnmodells eignet sich besonders gut als Ausgangsbasis für die Herstellung eines Zahnersatzteils, einer Zahnrestauration oder eines Zahnmodells, weil der generische Zahnmodelldatensatz im Gegensatz zu einem herkömmlichen elektronischen Zahnmodell nicht von mehr oder weniger mit der Natur übereinstimmenden Vorstellungen des Autors des elektronischen Zahnmodells basiert, sondern von reellen Zähnen bestimmt ist.

Wenn man beispielsweise eine Restauration für einen reparaturbedürftigen Zahn unter Zuhilfenahme eines erfindungsgemäß erhaltenen generischen Zahnmodells herstellt, gibt die in dem generischen Zahnmodell zum Ausdruck kommende natürliche Zahnform den Ausschlag und nicht ein menschlicher Vorstellung entsprungenes Zahnmodell.

Bei der Herstellung beispielsweise einer Zahnrestauration kann man das erfindungsgemäß erhaltene generische Zahnmodell als Ausgangsbasis nehmen und diesen Datensatz unter Anpassung an bestehen gebliebene Zahnflächenteile des reparaturbedürftigen Zahns oder der Restgebisssituation an den jeweils zu reparierenden Zahn anpassen, indem per interaktiven Eingriff oder durch softwaregesteuerte Automatik dieser Datensatz abgewandelt wird, um die genannte Anpassung an die Zahnflächenreste des zu reparierenden Zahns oder der den zu reparierenden Zahn umgebenden Restgebisssituation vorzunehmen.

Daher sieht das Verfahren gemäß Anspruch 1 vor, dass auf der Grundlage einer Korrespondenzanalyse eine Hauptachsenanalyse und eine Linearkombination in der in diesen Ansprüchen beschriebenen Weise durchgeführt werden, aus denen ein generischer Zahnmodelldatensatz erstellt wird. Mit Hilfe des generischen Zahnmodells lässt sich der Rahmen fest legen, innerhalb welchem eine Anpassung des Modelldatensatzes an das elektronische Bild der Reststruktur des zu reparierenden Zahns möglich ist, ohne sich aus dem Formenvorrat natürlicher Zahnformen weg zu bewegen. Die Anpassung des generischen Zahnmodelldatensatz an den zu reparierenden Teil des reparaturbedürftigen Zahns kann auf interaktive Weise oder auch vollautomatisch mittels Softwaresteuerung und -verarbeitung geschehen. Steuert man eine numerisch gesteuerte Maschine entsprechend einem auf diese Weise erhaltenen Datensatz, kommt man zu einem physischen Zahnteil, welches dem Aussehen der ehemaligen unversehrten Zahnfläche des zu reparierenden Zahns besonders gut nahe kommt, wobei man dieses Ergebnis auch auf für den Zahnmediziner oder Zahntechniker vergleichsweise einfache Weise erreichen kann.

Das Verfahren nach Patentanspruch 1 befasst sich mit der Schaffung eines generischen Zahnmodelldatensatzes eines bestimmten Zahntyps (z.B. OK-6er, oder auch großer, mittlerer und kleiner OK-6er, etc.). Diese Oberflächen erlauben schon für viele Situationen eine ausreichende zahnähnliche Rekonstruktion. Weiterhin ermöglicht der generische Zahnmodelldatensatz, das jede Veränderung, die unter gewissen Kriterien (siehe unten) an dieser Oberfläche durchgeführt wird, mit hoher Wahrscheinlichkeit wiederum eine natürliche Kaufläche ergibt und dass alle möglichen zugelassenen Varianten an Veränderungen die Gesamtheit nahezu aller in der Natur vorkommenden Zahnmorphologien beschreibt. Die Anzahl der Anpassungsvariablen ist dabei gering und die Rekonstruktion von Zahnoberflächen kann automatisiert werden.

Die Erzeugung dieses generischen Zahnmodelldatensatzes erfolgt dabei über eine möglichst große Anzahl von Datensätzen desselben Zahntyps. Im allgemeinen können die elektronischen Datensätze sowohl zwei- als auch dreidimensional vermessen sein. Zweidimensionale Vermessung geht z.B. mit der metrischen Photographie, dreidimensional z.B. mit Weißlicht-Streifenprojektion etc., auch Stereophotogrammetrische Verfahren wären denkbar. Zur Rekonstruktion von reparaturbedürftigen Zähnen und Defektsituationen benötigt man jedoch zumindest dreidimensional vermessene Datensätze. Beim Zahntyp kann es sich zum Beispiel um Molaren, Prämolaren, Eckzähne und Frontzähne handeln. Als Zahntyp kann aber auch der Oberkiefer(OK)-6er, Unterkiefer(UK)-4er, OK-1er etc. stehen. Möglich ist des weiteren eine Unterscheidung nach Alter und Abrasion, nach Geschlecht, nach Volkszugehörigkeit, nach Größe der Zähne, nach morphologischen Besonderheiten etc, z.B. können die Gruppen OK-7er im Alter von 50-60 Jahren, OK-6er mit und ohne Tuberculum Carabelli, UK-3er bei weiblichen Personen, Unterteilung in große, mittlere, kleine 6er etc. ein Beispiel für einen Zahntyp darstellen. Auch können z.B. benachbarte Zähne zu einem (kombinierten) Zahntyp zusammengefasst werden, um Zusammenhänge von Nachbarzähnen zu integrieren oder zu analysieren. Durch die Information des Nachbarzahnes könnte z.B. die Auswahl der Zahnoberfläche für den reparaturbedürftigen Zahn oder für die Defektsituation erfolgen. Der Begriff Zahntyp umfasst also eine je nach Aufgabenstellung sehr variable Gruppierungsmöglichkeit, was bei den Patentansprüchen in ihrer Allgemeinheit zu beachten ist.

Für einen bestimmten Zahntyp müssen die jeweiligen Datensätze in einem ersten Schritt zueinander referenziert werden (ins gleiche Koordinatensystem gebracht und ungefähr gleichmäßig ausgerichtet werden) und zwischen den Oberflächenpunkten des einen Datensatzes mit den jeweils anderen Datensätzen Korrespondenzen aufgebaut werden. Diese Korrespondenzen erfolgen z.B. zwischen markanten Punkten und Strukturen der Oberfläche. Diese Zuordnung kann manuell erfolgen, kann durch Suchen und Zuordnung von bestimmten charakteristischen Merkmalesstrukturen (Höckerform, Fissurenverlauf, Randleiste etc. ) erfolgen. Wahlweise ist hier ein Prozess vorzuziehen, der diese Korrespondenzpunkte und/oder -strukturen automatisch findet, da bis jetzt noch kein bewiesener metrisch erfassbarer Sachverhalt vorliegt, welche wirklich die markanten Punkte bzw. Strukturen oder Eigenschaften eines bestimmten Zahntyps sind. Im Gegenteil, es gibt bis jetzt in der gesamten zahnmedizinischen Fachliteratur keinen Hinweis auf eine nur annähernde mathematische Beschreibung von Zahnoberflächen, die in irgendeiner Weise für den CAD/CAM-Prozess geeignet wäre.

Als eine mögliche Implementierungsvariante hat sich folgendes Vorgehen als machbar erwiesen: Zuerst werden die Datensätze der vermessenen Zahnoberflächen eines bestimmten Zahntyps ins gleiche Koordinatensystem gebracht, um eine möglichst gute Ausgangsbasis für die automatische Korrespondenzpunktbestimmung zu bekommen. Dies kann mit Matching-Routinen durch Minimierung der Abstandsfehlerfunktion erfolgen, wobei Rotations- und Translationsparameter ermittelt werden. Nach erfolgter Koordinatentransformation findet die Korrespondenzanalyse statt. Aus der Bildverarbeitung können hier modifizierte Algorithmen zum optischen Fluss mit Erfolg angewandt werden. Weiterhin kann durch elastisches Registrieren bzw. Matchen von bestimmten Merkmalen (Fissuren, Höckerspitzen, Höckerabhängen, Randleisten) zwischen den einzelnen Zahnoberflächen Korrespondenzen gebildet und Abbildungsvorschriften gefunden werden. Am Schluss erhält man die Zuordnung vieler Punkte durch Korrespondenzen zwischen allen Datensätzen.

Genauer wird dies im Folgenden anhand des Verfahrens des optischen Flusses spezifiziert. Ausgangsbasis sind m Bibliothekszahnflächen eines bestimmten Zahntyps, entnommen einer Zahnbibliothek, in der Form $z_j(x,y)$, mit $j = 1,....m$ als Scandaten.

Genauso erlaubt sind parametrische Darstellungen $z_j(u,v)$, mit $u = u(x,y)$, $v = v(x,y)$. Dies können z.B. Polarkoordinaten etc. sein. Beliebige komplizierte dreidimensionale Oberflächen mit Unterschneidungen können stückweise durch obige Funktionen angenähert werden. Im erweiterten Sinne sind für andere Verfahren auch beliebig dimensionale Beschreibungen von Zähnen erlaubt.

Ausgehend von einem Referenzzahn $z_R(x,y)$, mit $R \in \{1,...,m\}$, wird mittels einer Korrespondenzanalyse zu jedem Punkt des Referenzzahnes der korrespondierende Punkt auf der Kaufläche $z_j(x,y)$ gesucht. Dies kann auch durch hintereinander geschaltetes Verknüpfen von Korrespondenzen erfolgen, indem von einem Zahn ausgehend die Korrespondenz zu einem weiteren Zahn aufgebaut wird, von diesem neuen Zahn eine weitere Korrespondenz zu einem dritten Zahn etc. Zusätzlich kann vor jeder neuen Korrespondenzermittlung auch ein neuer Durchschnittszahn aus den vorhanden Korrespondenzen berechnet und als Ausgangsbasis für die neue Korrespondenzanalyse dienen. Insgesamt kann dies durch einen Algorithmus erreicht werden, der diese Korrespondenzen automatisch ohne Vorkenntnisse auffindet, wie in Patentanspruch 6 spezifiziert. Eine Möglichkeit ist das Verfahren des optischen Flusses (für beliebige 3D-Objekte sind auch andere Möglichkeiten beschrieben in Shelton, C.R.: 3D Correspondence. Master Thesis, Massachusetts Institute of Technologie, 1998). Als Ergebnis erhält man ein zu jedem Zahn $z_j(x,y)$ gehöriges zweidimensionales Vektorfeld $\vec{v}_j(x,y)$ mit:

$$\vec{v}_j(x,y) = \begin{pmatrix} \Delta x_j(x,y) \\ \Delta y_j(x,y) \end{pmatrix}$$

so dass zu jedem Koordinatenpaar (x,y) des Referenzzahnes $z_R(x,y)$ sich der korrespondierende Punkt des Zahnes $z_j(x',y')$ aus der Beziehung:

$$z_j(x + \Delta x_j(x,y), y + \Delta y_j(x,y))$$

ergibt. Bei Zahnoberflächen ist es sinnvoll, neben der Glattheit des Verschiebungsfeldes bezüglich der z-Koordinaten, auch die Glattheit bezüglich der Steigungen zu fordern, da Steigungen auch ein wesentliches Merkmal der Kauflächen darstellen. Auch kann man bei der Vorgehensweise der Korrespondenzanalyse versuchen, nach jeder neuen Korrespondenzfindung diesen Datensatz zu den schon korrespondierenden Datensätzen hinzuzufügen und daraus eine neue Linearkombination suchen, die den nächsten Datensatz, der noch nicht korrespondiert, möglichst gut annähert. Diese neue Linearkombination kann dann zur automatischen Korrespondenzfindung herangezogen werden. So können iterativ alle Datensätze in Korrespondenz gebracht werden.

Nachdem nicht alle Punkte einer Oberfläche eindeutig den Punkten der anderen Oberfläche zugeordnet werden können, kann man fordern, dass sich das Verschiebungsfeld bildlich wie eine elastische Membran verhält: Zwischen den eindeutigen Korrespondenzen ist diese Membran nahezu nicht verschieblich, während sie dazwischen, d.h. in den Bereichen mit unklaren oder schwachen Korrespondenzen, sich nahezu frei entspannen kann. Berechnet werden kann dies zum Beispiel durch Minimierung einer Energiefunktion, die aus einer Kopplung von vielen Federn zwischen den einzelnen Oberflächenpunkten besteht (Näherung für die kontinuierliche elastische Membran).

Eine interessante Erweiterung für die Berechnung des optischen Flusses besteht darin, dass man neben der 3D-Datenrepräsentation $z(x,y)$ weitere Kriterien oder Oberflächenbeschreibungen für die Korrespondenzanalyse heranzieht, wie in Patentanspruch 3 für entsprechende elektronische Daten ausgewiesen. Dies könnte zum Beispiel das Gradientenfeld (Steigungen) der Zahnoberfläche sein. Gradienten beschreiben bestimmte Merkmale wie Kanten, Ecken oder stärkere Änderungen auf der Oberfläche besser als die reinen Höhendaten. Indem man nun einen neuen Merkmalsvektor $\vec{m}$ mit

$$\vec{m} = \begin{pmatrix} z(x,y) \\ \nabla z(x,y) \end{pmatrix}$$

bildet und eine neue Norm für diesen Merkmalsraum einführt:

$$\left\| \vec{m} \right\|^2 = z^2(x,y) + \beta \cdot (\nabla z(x,y))^2$$

wobei $\beta$ die Gewichtung des Gradientenfeldes im Verhältnis zum Höhenbild festlegt, so kann das Verschiebungsfeld $\vec{v}(x,y) = (\Delta x(x,y), \Delta y(x,y))^T$ für den Merkmalsvektor $\vec{m}$ analog zu oben berechnet werden, wenn man die Normen $\|\vec{m}_x^2\|$ und $\|\vec{m}_y\|^2$ und die jeweiligen Skalarprodukte $\langle \vec{m}_x, \vec{m}_y \rangle$ bzw. $\langle \vec{m}_x, \Delta\vec{m} \rangle$ verwendet. Natürlich könnte man sich auch mehrdimensionale Merkmalsvektoren vorstellen, indem man noch weitere Eigenschaften der Zahnoberfläche mit berücksichtigt. Dies könnten zum Beispiel Texturwerte, Krümmungen etc. sein. Der Gewichtungsfaktor $\beta$ (oder weitere Gewichtungsfaktoren) erlaubt die Möglichkeit, den jeweiligen Einfluss der einzelnen Merkmalsfelder festzulegen. Mit all diesen Maßnahmen liegt ein starkes Werkzeug vor, dass für die Zahnoberflächen eine automatische, ohne Vorkenntnisse erfordernde Analyse von Korrespondenzen ermöglicht.

Sind diese Zuordnungen gefunden, kann in einem nächsten Schritt der Referenzzahn als Vektor in einem 3n-dimensionalen Raum repräsentiert werden (n ist dabei die Anzahl der ausgewählten auf der Zahnoberfläche liegenden Punkte, idealerweise wird man ein äquidistantes Gitter zugrundelegen, die typische Anzahl von Punkten kann von 10000-200000 gehen):

$$\vec{D}_R = (x_1, y_1, z_R(x_1, y_1), x_2, y_2, z_R(x_2, y_2), \ldots, x_n, y_n, z_R(x_n, y_n))$$

In konsistenter Weise können dann ausgehend vom Referenzzahn (oder der Linearkombination) und dem entsprechenden Vektorfeld $\vec{v}_j(x,y)$ alle anderen Zähne der Bibliothek als 3n-dimensionaler Vektor dargestellt werden:

$$\begin{aligned} \vec{D}_j = (&x_1 + \Delta x_j(x_1, y_1),\ y_1 + \Delta y_j(x_1, y_1),\ z_j(x_1 + \Delta x_j(x_1, y_1), y_1 + \Delta y_j(x_1, y_1)), \\ &x_2 + \Delta x_j(x_2, y_2),\ y_2 + \Delta y_j(x_2, y_2),\ z_j(x_2 + \Delta x_j(x_2, y_2), y_2 + \Delta y_j(x_2, y_2)), \ldots, \\ &x_n + \Delta x_j(x_n, y_n),\ y_n + \Delta y_j(x_n, y_n),\ z_j(x_n + \Delta x_j(x_n, y_n), y_n + \Delta y_j(x_n, y_n))) \end{aligned}$$

Auf diese Weise repräsentieren gleiche Vektorkoordinaten, d.h. Indizes, auch jeweils korrespondierende Punkte und zwar zwischen allen Zähnen. Die Gesamtheit der m Vektoren, die den m Bibliothekszähnen entsprechen, spannen einen Raum auf, den man als den Zahnraum für den entsprechenden Zahntyp bezeichnet. Damit lässt sich beispielsweise auch ein Durchschnittszahn $\vec{D}$ aus den einzelnen umgeformten Bibliothekszähnen $\vec{D}_j$ berechnen:

$$\vec{D} = \frac{1}{m} \cdot \sum_{j=1}^{m} \vec{D}_j$$

Man kann an dieser Stelle den neuen Durchschnittszahn wiederum als Referenzzahn verwenden und obigen Prozess nochmals neu starten und auch öfters wiederholen. Damit kann der Durchschnittszahn noch allgemeiner bestimmt werden. Oder man nimmt verschiedene Referenzzähne und mittelt nachher das Ergebnis. Im Patentanspruch 2 wird dieser Durchschnittsdatensatz als Durchschnittszahn einer bestimmten Zahngruppe (Zahntyp) verwendet.

Liegen die einzelnen Zahnoberflächen als Vektoren vor, ist es mit hoher Wahrscheinlichkeit möglich, jeden neu hinzugekommenen Zahn $\vec{Z}$ als Linearkombination der vorhandene Zähne zu repräsentieren:

$$\vec{Z} \approx \sum_{j=1}^{m} \beta_j \cdot \vec{D}_j$$

Um die Anzahl der Linearfaktoren $\beta_j$ und der Zähne $\vec{D}_j$ zu reduzieren, bietet sich die Hauptachsenanalyse an. Nachdem jeder Zahntyp für den Experten durch bestimmte Merkmale erkennbar ist, sollten durch die Hauptachsentransformation auch diejenigen Komponenten großen Einfluss haben, die bestimmte Merkmale des Zahntyps charakterisieren. So erhält man durch die Linearkombination eines Teils der Hauptachsen eine ausreichende Beschreibung der meisten Zahnoberflächen. Diese Hauptachsenanalyse kann auf den Zahndaten $\vec{D}_j$ direkt durchgeführt werden, wie im Patentanspruch 1 ausgeführt. Der verwendete Anteil p der sich ergebenden Hauptachsen (in der Regel die, die am meisten zur Varianz beitragen) wird durch Linearkombination (Linearfaktoren $a_i$ und Hauptkomponenten $\vec{P}_i$) mathematisch wie folgt verknüpft:

$$\vec{Z} \approx \sum_{l=1}^{p} a_l \cdot \vec{P}_l \qquad \text{(Gl. 1)}$$

Wie im Patentanspruch 2 ausgeführt, kann man, bevor die Hauptachsenanalyse bei den Zahnvektoren durchgeführt wird, den Vektorraum so verschieben, das der Mittelwert 0 ergibt. Dies erhält man durch Differenzbildung zwischen den einzelnen Zahnvektoren und dem Durchschnittszahn. Die entstandenen Differenzvektoren können dann ebenfalls durch Hauptkomponentenverfahren analysiert werden. Insgesamt bekommt man durch diese Verfahren mit wenigen veränderbaren Parametern eine ausreichend effiziente Beschreibung neuer Zahnformen, die sich als Linearkombinationen dieser neuen Parameter (Linearfaktoren) und Hauptachsen darstellen lassen. Der entscheidende Vorteil ist, dass bei Veränderung der Parameter mit hoher Wahrscheinlichkeit einer der vorhandenen natürlichen Zahndaten angenähert wird. Die anzufertigende Restauration wird also zahnähnlich sein und die Gefahr unschöner Kauflächen ist gebannt.

Im folgenden wird die Hauptachsenanalyse bei den Zahnvektoren für den Fall genauer beschrieben, für den der Durchschnittszahn subtrahiert wird, d.h. der Vektorraum der Zähne so verschoben wird, dass der Mittelwert 0 ergibt. Damit sind auch nach der Hauptachsenanalyse die Mittelwerte der Hauptachsen (Eigenvektoren) 0. Es wird von jedem Zahnvektor $\vec{D}_j$ der Durchschnittszahn $\vec{D}$ abgezogen und ein neuer Differenzvektor $\vec{\Delta}_j$ gebildet:

$$\vec{\Delta}_j = \vec{D}_j - \vec{D}$$

Die Hauptachsenanalyse liefert dann die Eigenwerte $\lambda_k$ mit den zugehörigen Hauptachsen (Hauptkomponenten, Eigenvektoren) $\vec{P}_k$, k=1,...m. Folgende Eigenschaften ergeben sich:

1. Die Eigenwerte $\lambda_k$ entsprechen den Varianzen in Richtung der Hauptachse $\vec{P}_k$

2. Die Summe der Eigenwerte $\lambda_k$ entspricht der Summe de Varianzen von $\vec{\Delta}_j$, also der gesamten Varianz von $\Delta_j$. Nachdem eine Mittelwertverschiebung keinen Einfluss auf die Varianz der Werte hat, entspricht daher die Summe der Eigenwerte $\lambda_k$ der Gesamtvarianz von $\vec{D}_j$.

3. Der Anteil einer Hauptkomponente $\vec{P}_k$ an der Gesamtvarianz der Datensätze ist gegeben durch:

$$\lambda_k \Big/ \sum_{l=1}^{m} \lambda_l$$

4. Der Anteil der ersten p Hauptkomponenten $\vec{P}_k$ an der Gesamtvarianz ist analog gegeben durch:

$$\sum_{l=1}^{p} \lambda_l \Big/ \sum_{l=1}^{m} \lambda_l$$

Bei den Oberkiefermolaren zeigt sich zum Beispiel, dass die ersten 7 Hauptkomponenten ca. 70% der Gesamtvarianz von 170 Zähnen beschreiben.

Ein Großteil aller möglichen Zahnoberflächen $\vec{Z}$ lässt sich nun relativ genau annähern durch eine Linearkombination der ersten p Hauptkomponenten $\vec{P}_k$ ($\alpha_i$ sind die Linearfaktoren):

$$\vec{Z} \approx \vec{D} + \sum_{l=1}^{p} \alpha_l \cdot \vec{P}_l \qquad\qquad \text{(Gl. 2)}$$

Wenn man sinnvolle Randbedingungen an die Parameter $\alpha_i$ (Gl. 1) bzw. $\alpha_i$ (Gl. 2) stellt (z.B. dass der neue Zahn sich innerhalb des von den vorhandenen Zähnen aufgespannten Raumes befindet oder zumindest nicht allzu weit entfernt liegen sollte), wird jede beliebige Linearkombination nach (Gl. 1) oder (Gl. 2) wiederum einen Zahn beschreiben. Ein Zahndatensatz, der allgemein durch eine Linearkombination von Hauptachsen und evtl. Addition von Durchschnittszahn erzeugt wird, wird in dieser Patentschrift als generischer Zahnmodelldatensatz oder als generisches Zahnmodell bezüglich des betrachteten Zahntyps bezeichnet. Synonym dazu und im abstrakten Sinne wird ebenfalls in dieser Patentschrift der generische Zahnmodelldatensatz oder das generische Zahnmodell bezüglich des betrachteten Zahntyps aufgefasst als eine Kombination von Datensätzen der ausgewählten Hauptachsen und evtl. dem Durchschnittszahn. Diese Kombination kann man sich physisch z.B. vorstellen entweder als einzelne Datensätze, die durch Verkrüpfungen oder Verweise verbunden sind, oder durch Zusammenfügung zu einem großen Datensatz. Wird nun eine Repräsentation dieses generischen Zahnmodells oder generischen Zahnmodelldatensatzes gewünscht, müssen nur die speziellen Linearfaktoren mit den Hauptachsen multipliziert und evtl. der Durchschnittszahn addiert werden. Das generische Zahnmodell oder der generische Zahnmodelldatensatz (im folgenden zum Teil auch als "generischer Zahn" abgekürzt) stellt also eine Art mathematische Beschreibung des gesamten Zahnraumes des entsprechenden Zahntyps dar.

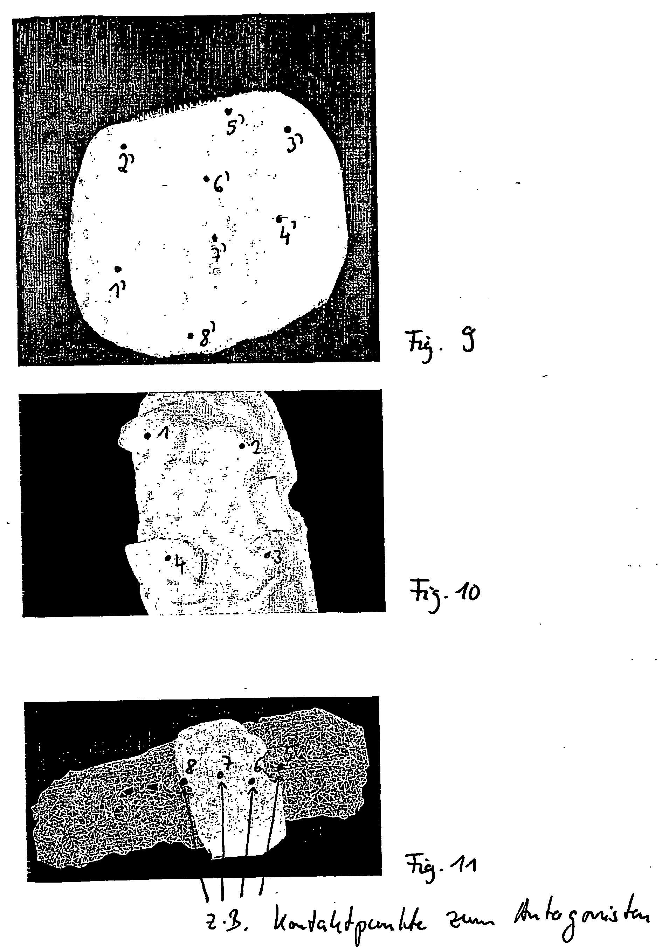

Der Rekonstruktionsvorgang für den reparaturbedürftigen Zahn bzw. die Defektsituation lässt sich mittels des generischen Zahnmodells durchführen und auch weitgehend automatisieren. Rekonstruktion bedeutet die vollständige oder zumindest teilweise Wiederherstellung der fehlenden Außenhülle des reparaturbedürftigen Zahnes oder der Defektsituation. Beim reparaturbedürftigen Zahn kann es sich um Inlay-, Onlay-, Overlay-, Teilkronen-, Kronen-, Brückenpräparationen etc. handeln, bei der Defektsituation geht es um das Auffüllen von Bereichen mit fehlenden Zähnen, z.B. Brückenzwischenglieder, Implantataufbauten oder Teilen von Teilprothesen bzw. Totalprothesen. Bei dem Begriff der Restgebisssituation handelt es sich in dieser Patentschrift um die vermessene Information (insbesondere Datensätze) von präpariertem Zahn oder Zähnen (reparaturbedürftiger Zahn oder Zähne) oder Defektsituationen und die zusätzliche wahlweise Einbeziehung von vermessener Information der Restzahnsubstanz, des Gegenkiefer, des funktionellen und statischen/okklusalen Bissregistrats, des Nachbarzahns/ der Nachbarzähne und/oder des Zahnfleischanteils bzw. des Kieferkamms. Beim Gegenkiefer ist in der Regel nur die Einbeziehung von einem oder mehreren Gegenzähnen, d.h. dem Zahn oder der Zähne, die dem reparaturbedürftigen Zahn oder der Defektsituation gegenüberliegen, ausreichend. Der Begriff Gegenzahn ist synonym zum Fachbegriff Antagonist zu stehen. In dieser Patentschrift werden jedoch zusätzlich unter dem Begriff Gegenzahn auch Teile des Gegenkiefers oder der gesamte Gegenkiefer subsumiert. Wählt man an der betreffenden Präparation bzw. Defektsituation und umgebender Restgebisssituation bestimmte Konstruktionspunkte bzw. Korrespondenzpunkte oder Korrespondenzstrukturen, z.B. Höckerspitzen oder Randleistenpunkte auf Restzahnsubstanz und/oder mögliche Kontaktpunkte mit dem Gegenzahn oder Nachbarzahn (Fig. 9 - 11), so kann bei Kenntnis der entsprechenden Korrespondenzpunkte und -Strukturen am generischen Zahnmodell, Durchschnittszahn etc. die Rekonstruktion bestmöglich durch Optimierungsprozesse durchgeführt werden. Beim Durchschnittszahn werden in der Regel Rotations-Translations, Skalierungs- und evtl. auch affine Transformationsparameter durch Minimierungsprozesse ermittelt. Beim generischen Zahn erfolgt zusätzlich noch die optimierte Anpassung der Parameter (Linearfaktoren) der Hauptachsen so, dass das Einfügen des generischen Zahnes, der entsprechend der Parameter verändert wurde, bestmöglich erfolgt. Wahlweise können in diesem Prozess auch Nebenbedingungen eingebaut werden, wie z.B. Begrenzung der Größe der Parameter, damit das Ergebnis nicht weit außerhalb des Zahnraumes liegt, oder die Bedingung, dass die Gegenkaufläche oder das funktionelle Registrat nicht durchdrungen werden darf, an den Kontaktpunkten sich a-ber berührt. Auch können Qualitätsparameter wie minimale Schichtstärken für ein Material oder belastungsoptimiertes Oberflächendesign mit berücksichtigt werden.

Neben den einzelnen Korrespondenzpunkten können aber auch alle vorhandenen Restzahnflächen (z.B. bei Inlays, Onlays, Teilkronen), oder auch Korrespondenzstrukturen, d.h. bestimmte charakteristische Areale und Formen, in ihrer Gesamtheit hergenommen und alle Punkte dieser Restzahnflächen und/oder Strukturen zur Korrespondenz genommen werden. Dies kann z.B. analog zu oben wiederum mit dem Verfahren des optischen Flusses durchgeführt werden. Eine andere Möglichkeit ist das Matching mit Optimierung der Parameter entsprechend einer Gütefunktion (z.B. Abstandsfunktion). Entscheidend dabei ist wiederum, das der Zahn nicht irgendwie deformiert wird, sondern entlang der Hauptachsen und damit im Bereich der Form natürlicher Zähne bleibt.

Im allgemeinen werden die generischen Kauflächen und Datensätze der Defektsituation bzw. des reparaturbedürftigen Zahns nicht im gleichen Koordinatensystem liegen. Daher müssen bei der generischen Kaufläche neben den Parametern entlang der Hauptachsen (Linearfaktoren) zumindest auch die Rotation und Translation bestimmt werden. Die Einbeziehung einer Skalierung ist möglich, aber hier nicht unbedingt sinnvoll, da dieser Faktor bereits in der Hauptachsenrepräsentation integriert sein sollte. Eine Möglichkeit der Problemlösung besteht darin, den Anpassungsprozess in zwei Stufen zu durchlaufen:

1. Rotation und Translation des Durchschnittszahnes in das Koordinatensystem des Defektzahnes anhand der Korrespondenzpunkte und/oder Restzahnsubstanz. Dies kann mit z.B. dem Algoritmus nach Umeyama (Umeyama S.: Least-squares estimation of transformation parameters between two point patterns. IEEE PAMI 13(4); 276-280, 1991) durchgeführt werden, wobei man den Skalierungsfaktor gleich 1 setzt.

2. Verbesserung der Anpassung der Korrespondenzpunkte durch Optimierung der Hauptachsenparameter (evtl. ergänzt durch lineare Faktoren der Rotation und Translation etc.).

Dadurch kann man für beide Stufen direkte Lösungsverfahren einsetzen. Im allgemeinen Fall (auch einstufige Lösung) können natürlich auch bekannte nichtlineare iterative Lösungsverfahren eingesetzt werden (z.B. Gradientenabstiegsmethode, Levenberg-Marquardt etc.).

Wurde der ursprüngliche Datensatz der Restzahnsubstanz und/oder Korrespondenzpunkte in das Koordinatensystem des Durchschnittszahnes translatiert und rotiert, bestehen mit den Eigenschaften der generischen Zahnoberfläche bestmögliche Ausgangsbedingungen für die Rekonstruktion von Zahnoberflächen. Die Aufgabe besteht darin, die Parameter (Linearfaktoren) $\alpha_i$ so zu bestimmen, dass sich die sich ergebende Linearkombination (d.h. neue Kaufläche) möglichst gut an die vorhandene Situation anpasst. Dies erfolgt z.B. durch Minimierung einer Fehlerfunktion.

Eine weitere Optimierung der Anpassung besteht darin, nur solche Linearkombinationen zuzulassen, die eine sehr hohe Wahrscheinlichkeit aufweisen, d.h. die möglichst typische Zahnformen des Zahnraums bevorzugen. Damit soll das Ergebnis mit hoher Wahrscheinlichkeit in der konvexen Hülle der Zahndaten liegen. Es ist in diesem Zusammenhang alternativ denkbar, eine wahrscheinlichkeitstheoretische Betrachtung mit einzubeziehen. Folgende Bedingungen sollten berücksichtigt werden:

a) Die gesuchte Kaufläche sollte im Raum der Zahnoberflächen eine möglichst hohe Wahrscheinlichkeit aufweisen, d.h. ihre Form sollte möglichst typisch für eine Kaufläche sein.
b) Die gemessenen Punkte können Messfehler aufweisen (z.B. durch Messung oder durch Anklicken). Damit ein Mess- oder Bearbeitungsfehler bei der Auswahl der Kaufläche nicht übermäßig gewichtet wird, wird man auch hier eine Wahrscheinlichkeit für einen Messpunkt, abhängig vom Rauschen oder Fehlerquellen, berücksichtigen.

Ein solcher Ansatz könnte zu folgender Maximierung der Wahrscheinlichkeit führen:

$$P(\vec{c} \mid \vec{z}_{real}) = const \cdot P(\vec{z}_{real} \mid \vec{c}) \cdot P(\vec{c})$$
$$= const \cdot e^{-\frac{1}{2\sigma^2}\|\mathbf{M}\vec{c}-\vec{z}_{real}\|^2} \cdot e^{-\frac{1}{2}\|\vec{c}\|^2}$$

Diese Wahrscheinlichkeit wird maximal, wenn die Gütefunktion E minimal wird:

$$E = \left\| \mathbf{M}\vec{c} - \vec{z}_{real} \right\|^2 + \gamma \cdot \left\| \vec{c} \right\|^2 = \min, \qquad \gamma = \frac{1}{\sigma^2}$$

mit

$$\vec{z} = \sum_{l=1}^{p} \lambda_l c_l \vec{p}_l = \mathbf{M}\vec{c},$$

mit der Matrix M = $(\lambda_1\vec{p}_1, \lambda_2\vec{p}_2, ..., \lambda_p\vec{p}_p)$, und der Messfehler mit einer Varianz von $\sigma^2$.

Die ermittelte optimale generische Zahnoberfläche wird sich schon sehr gut in die gegebene Restgebisssituation einfügen. Bei der Restgebisssituation handelt es sich um die vermessene Information (insbesondere Datensätze) von präpariertem Zahn inkl. Restzahnsubstanz, Gegenkiefer, funktionellem und statischem Bissregistrat, Nachbarzähnen und/oder auch vom Zahnfleischverlauf und Kieferkamm. Allerdings werden sich in der Regel noch kleinere Differenzen ergeben, wie z.B. kleine Stufen oder Lücken beim Übergang zur Restzahnsubstanz, zu hohe Stellen, die das Bissregistrat oder den Nachbarzahn durchdringen, noch fehlende Kontaktpunkte etc. Außerdem müssen unter Umständen noch fehlende Flächenanteile wie Approximalflächen, Oral- und Vestibulärflächen ergänzt werden. Diese zusammengefasst als Anpassung bezeichneten Vorgänge, die meistens nur geringfügige Änderungen beinhalten, liefern dann den fertigen Datensatz, der für die Steuerung einer Maschine benutzt wird.

In Patentanspruch 5 wird die Verwendung dieser ermittelten Datensätze für die physische Herstellung beschrieben. Im Prinzip sind alle möglichen automatisierten

Fertigungsverfahren wie CNC-Fräsen oder -schleifen, Laserbearbeitung, Stereolithographie oder lithographische Sinterverfahren einsetzbar. Die Materialpalette für die Zahnrestauration, Zahnersatzteile oder Zahnmodelle kann von Kunststoff über Metalle (Titan, Gold, Stahl etc.) bis zu Keramik reichen. In der Zahnmedizin sind bereits eine Reihe von Materialien speziell für den CAD/CAM-Prozess verfügbar.

Patentanspruch 5 beinhaltet den gesamten Herstellungsprozess von Vermessung bis Produktion. Oben aufgeführte Implementierungsvarianten können hier analog eingesetzt werden. Aus der Beschreibung und den Zeichnungen kann ein Fachmann weitere Varianten, die hier nicht einzeln aufgeführt sind, ableiten, so dass auch diese in die Patentschrift als vollumfänglich einbezogen betrachtet werden können.

Patentanspruch 6 betrifft ein Verfahren zur Herstellung eines dreidimensionalen elektronischen Datensatzes von Zahnersatzteilen oder Zahnrestaurationen, bei welchem nach erfolgter Zuordnung von Korrespondenzpunkten und/oder -strukturen des reparaturbedürftigen Zahnes und/oder der Defektsituation zum generischen Zahnmodelldatensatz die Linearfaktoren für den verwendeten Teil der Hauptachsen so optimiert werden, dass die neue Linearkombination möglichst gut die Korrespondenzen anpasst oder zur Deckung bringt.

Patentanspruch 7 betrifft ein Verfahren, bei welchem die Linearfaktoren durch Minimierung der Abstände zwischen den Korrespondenzpunkten ermittelt werden.

Patentanspruch 8 betrifft ein Verfahren, bei welchem die Linearfaktoren so ermittelt werden, dass die Wahrscheinlichkeit für die ermittelte Linearkombination möglichst hoch ist.

Patentanspruch 9 betrifft ein Verfahren, bei welchem die Optimierung die Wichtigkeit bestimmter Korrespondenzpunkte und/oder Korrespondenzstrukturen in Form von Wichtungsfaktoren berücksichtigt.

Patentanspruch 10 bezieht sich explizit auf die Berücksichtigung von funktionellen und/oder statischen bzw. okklusalen Bissregistraten. Ein großer Vorteil der gesamten Kauflächenanpassung mittels mathematischer bzw. elektronischer Vorgehensweise besteht darin, dass man die gesamte Herstellungskette von Gegenkieferabformung, Herstellung eines Gipsmodells dieses Gegenkiefers, Einartikulation des Gegenkiefers und Zuordnung zum gesägten oder Präparationsmodell, bis zur Bestimmung und Justierung der Kiefergelenksparameter etc. nicht mehr durchführen muss. Die Alternative stellt hier die direkte Abformung der Gegenkiefersituation mittels Bissregistrat im Mund dar. Das statische Bissregistrat, manchmal auch als okklusales Bissregistrat bezeichnet, erhält man durch Einbringung von Abformmaterial an die gewünschte Stelle, indem der Patient zubeißt und die Zähne zugebissen lässt, bis das Material abbindet. Über die Kieferbewegungen erhält man Auskunft, indem vor dem Abbindens des eingebrachten Abdruckmaterials der Patient zusätzlich noch möglichst viele verschiedene Kieferbewegungen ausführt. Dies ergibt dann das funktionelle Bissregistrat, manchmal auch als FGP (function generated path) bezeichnet. Mit dieser Vorgehensweise erhält man sehr genaue 3D-Informationen über die Bahnen des der Präparation gegenüberliegenden Zähnen, und damit auch Begrenzungslinien und Konstruktionshinweise, wo Kontaktpunkte liegen könnten und wo die rekonstruierte Zahnoberfläche nicht ausgedehnt werden darf, bzw. welches die höchsten Stellen sein können. In Patentanspruch 11 wird genau diese Information für die Korrespondenzfindung und damit für eine genauere Adaptation des Durchschnittszahnes oder des generischen Zahnes herangezogen. Durch geeignete mathematische Formulierung kann diese Information in Form von Randbedingungen in die Optimierungs- bzw. Minimierungsverfahren einbezogen werden. Diese Bedingung könnte z.B. lauten: Kontaktpunkte sind Berührungspunkte (Interpolation des Punktes mit zweiter Ableitung gleich 0) mit dem Bissregistrat, während die restlichen Bereiche von der rekonstruierten Fläche nicht berührt werden dürfen.

In Patentanspruch 12 wird eine Möglichkeit der Automatisierung der Kontaktpunktfindung mit dem Gegenzahn (Antagonisten) beschrieben. Durch Vergleich des statischen (okklusalen) Bissregistrats mit dem funktionellen Bissregistrat, die beide für die entsprechende Situation wie oben ausgeführt vom Patienten genommen wurden und sich als vermessene Datensätze im gleichen Koordinatensystem befinden (referenziert sind), sind die Bereiche, bei denen das eine Bissregistrat einen geringen Abstand von dem anderen Bissregistrat hat oder sich beide berühren, besonders ausgezeichnet. Diese Areale stellen die möglichen Kandidaten für die Kontakte mit den Antagonisten dar, in den anderen Bereichen können keine Kontaktpunkte liegen. Wenn man weiß, wo sich die korrespondierenden Kontaktpunkte auf der generischen Zahnoberfläche bzw. auf dem Durchschnittszahn befinden, kann man die Optimierung der Linearfaktoren weitestgehend automatisieren.

In Patentanspruch 13 werden zusätzlich für die Approximalflächengestaltung (z.B. Lage des Approximalkontaktes, Ausdehnung etc.) und für die Auswahl von Korrespondenzpunkten bzw. -strukturen (z.B. Randleisten, Formen der Kaufläche etc.) die vermessenen Informationen der Nachbarzähne einbezogen. Ebenso können einzelne Punkte (z.B. Kontaktpunkte) oder die Form und Strukturen des Gegenzahnes für die Korrespondenzbildung ausgenutzt und damit die Auswahl der bestpassendsten Zahnoberfläche für die Rekonstruktion des reparaturbedürftigen Zahnes bzw. Defektsituation durchgeführt werden. Ebenso könnte die Information des entsprechenden symmetrisch gegenüberliegenden Zahnes herangezogen werden, da man oft davon ausgeht, dass diese Zahnformen nur spiegelbildlich sind, sich aber sonst sehr stark ähneln. Insbesondere beinhaltet dieser Anspruch die Möglichkeit, die aus der Hauptachsenanalyse bzw. Korrespondenzanalyse gefundenen Zusammenhänge zwischen Nachbarzähnen des gleichen Patienten (z.B. bei der Herstellung des generischen Zahnmodells von benachbarten Zähnen) aus der Information des Nachbarzahnes/ der Nachbarzähne auf die

zu ergänzende Außenhülle oder zumindest Teile dieser Außenhülle zu schließen. Eine Möglichkeit besteht in der Optimierung der Parameter des kombinierten generischen Zahnmodelldatensatzes bei der Anpassung an den Nachbarzahn/Nachbarzähne, wobei gleichzeitig die zu rekonstruierende Zahnoberfläche entsprechend verändert wird. Das gleiche Verfahren ist auch für den Gegenzahn bzw. den symmetrisch gegenüberliegenden Zahn anwendbar. Insbesondere wird in diesem Anspruch auch darauf hingewiesen, dass die Information von Nachbarzahn/Nachbarzähnen, von Gegenzahn und/oder vom symmetrisch gegenüberliegenden Zahn/Zähnen auch aus zweidimensional vermessenen Datensätzen bestehen kann. Ausgehend von diesen Datensätzen kann man unter Zuhilfenahme eines entsprechenden generischen Zahnmodells durch Optimierung von Abbildungs-, Beleuchtungs-, Render- und/oder Projektionsfunktionen auf die dreidimensionale Struktur schließen (siehe z.B. Blanz, V., Romdhani, S.: Face Identification across different poses and illuminations with a 3D morphable model. Proc. Int. Conference on Automatic Face and Gesture Recognition, 202-207, 2002) und diese wiederum für die Rekonstruktion ausnutzen. Der Vorteil dieser zweidimensionalen Vermessung liegt darin, dass man relativ einfach z.B. mittels einer intraoralen Kamera oder eines Fotoapparats am Patienten diese Aufnahme bzw. Datensätze erstellen kann.

Weiterhin ist es notwendig Anpassungen durchzuführen, sofern noch Störstellen und Interferenzen nach Berechnung des bestpassendsten generischen Zahnes oder Durchschnittszahnes vorhanden sind. Dies können kleine Stufen oder Lücken beim Übergang zur Restzahnsubstanz, zu hohe Stellen, die das Bissregistrat oder den Nachbarzahn durchdringen, noch fehlende Kontaktpunkte etc sein. Hier bieten sich Verfahren an, die gewährleisten, dass die Veränderungen lokal begrenzt und möglichst klein bleiben und gleichzeitig einen harmonischen und glatten Übergang zu den nicht veränderten Bereichen ergeben. Dies kann durch bekannte Deformations und/oder Morphing-Verfahren erfolgen. Außerdem müssen unter Umständen noch fehlende Flächenanteile wie Approximalflächen, Oral- und Vestibulärflächen ergänzt werden. Die möglichen Verfahren einer automatisierten Ergänzung dieser Flächen werden weiter unten beschrieben. Insgesamt können diese Prozesse automatisch oder interaktiv erfolgen. Bei der interaktiven Manipulation kann der Zahnarzt oder Zahntechniker nach seiner jeweiligen Vorstellung die Gestaltung noch optimieren. Diese Möglichkeit sollte in Verfahren zur Herstellung von Zahnersatzteilen oder Zahnrestaurationen in der Regel immer implementiert sein. Mit Hilfe des generischen Zahnes gelingt die Verwirklichung verschiedener Okklusions- und Funktionskonzepte. In der Zahntechnik gibt es verschieden Theorien, wo statische und funktionelle Kontaktpunkte zum Nachbarzahn oder Antagonisten liegen sollen. Der generische Zahn bietet die Möglichkeit, quasi online zu entscheiden, welches Konzept man verwenden möchte und wo die Kontaktpunkte liegen sollten (Fig. 9-11). Dabei wird z.B. entweder einmal für einen bestimmten Benutzer oder Labor, der oder das ein bestimmtes Konzept favorisiert, oder auch vor jeder neuen Versorgung die gewünschten Kontaktpunkte auf dem generischen Zahn markiert, die entsprechenden Korrespondenzpunkte auf dem Bissregistrat und/oder der Restzahnsubstanz bzw. dem Nachbarzahn. Durch Anpassung der Parameter bezüglich der korrespondierenden Punkte erhält man nach Minimierungsverfahren wiederum eine funktionell gestaltete natürliche Kaufläche. Dieses Verfahren geht nur mit generischen Zähnen, da im Falle von Zahnbibliotheken der beste Zahn nur ausgewählt werden kann, wenn bei Veränderung der Kontakt/Funktionssituation die entsprechenden Referenzpunkte aller Zähne neu bestimmt werden müssen, bei hoher Anzahl von Zähnen ein aufwendiges Unterfangen. Auf der anderen Seite ist bei Deformation nur eines Modellzahnes, der nicht auf Basis eines generischen Zahnes erzeugt ist und die Hauptkomponentenanalyse nicht durchgeführt wurde, nicht gewährleistet, dass das Ergebnis ein harmonisches, zahnähnliches Resultat ergibt.

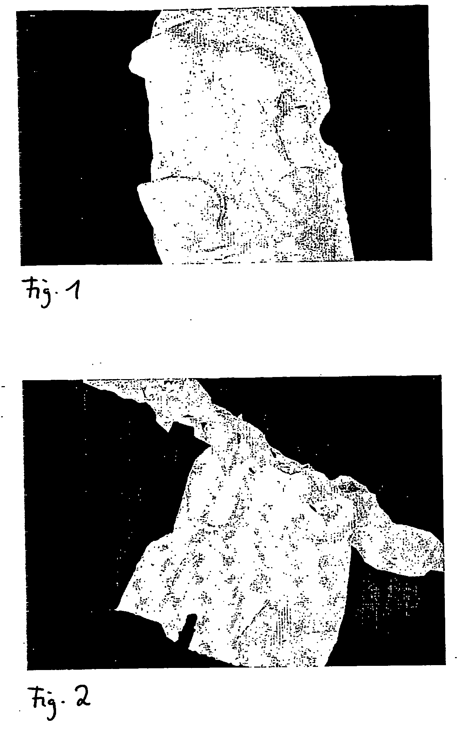

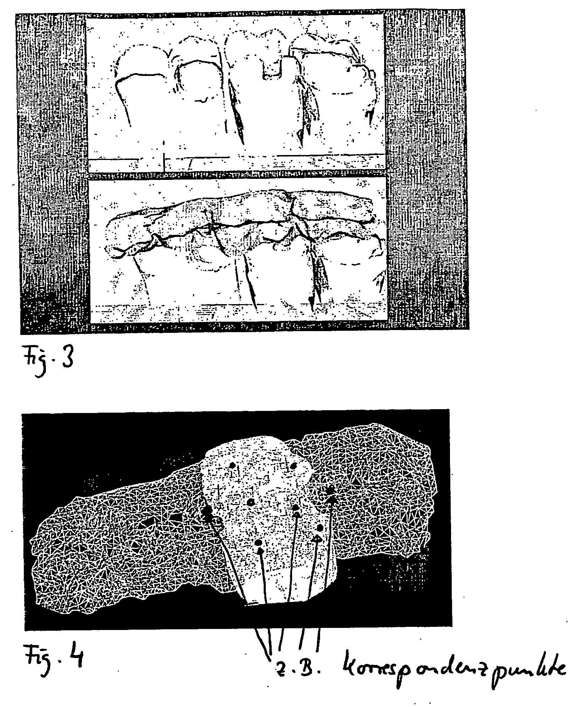

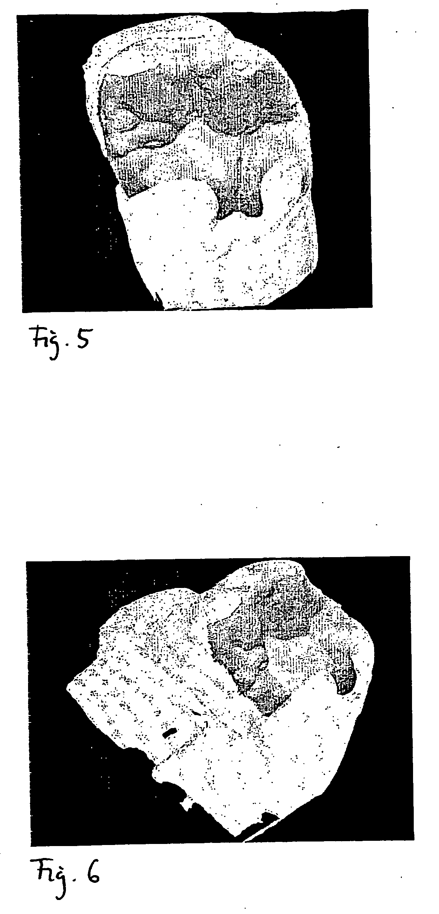

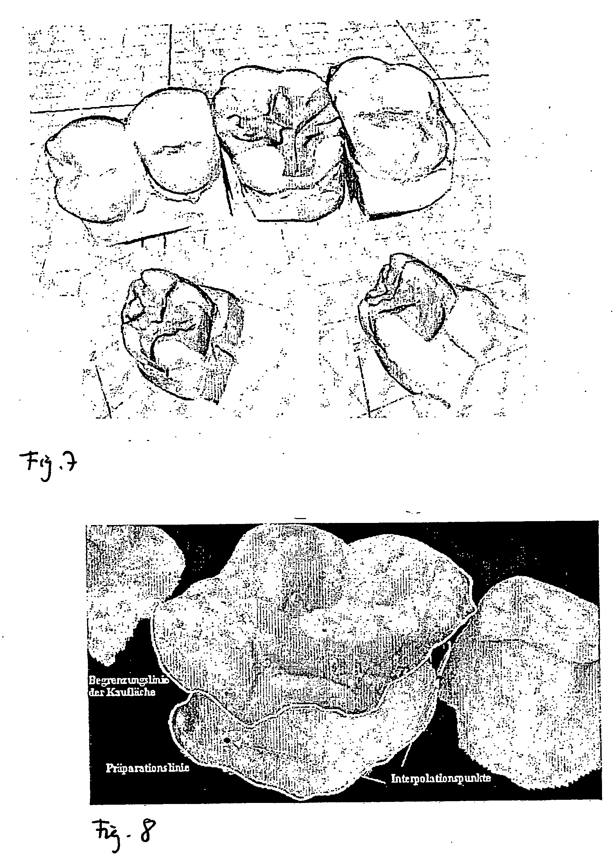

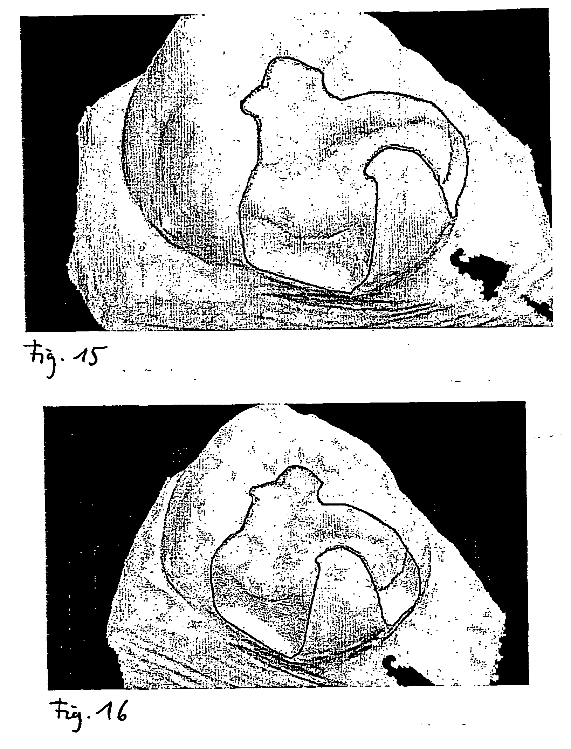

Es ist auch möglich Zahnersatzteile, die ausgehend von 3D-Datensätzen der Gegenkiefersituation (Fig.2) und der Präparation (Fig. 1) bzw. mehreren Präparationen, die zueinander referenziert sind, dadurch herzustellen, dass nach Referenzierung die vorhandenen Bissregistrate mit den Päparationsdatensätzen anhand der möglichen Überlappbereiche (Fig. 3) nach Auswahl geeigneter Korrespondenzpunkte (Fig. 4) die am besten passende Kaufläche aus einer Zahnbibliothek automatisch ausgewählt wird (Fig. 5). Ein Fehler-Minimierungs-Verfahren für die Auswahl und Anpassung der Bibliothekskaufläche, das sich hierfür sehr gut eignet und nicht iterativ erfolgt, wird z.B. bei Umeyama (Umeyama S.: Least-squares estimation of transformation parameters between two point patterns. IEEE PAMI 13(4): 276-280, 1991) beschrieben. Anschließend werden vorhandene Interferenzen bzw. Überschneidungen mit der Gegenzahnreihe und /oder Nachbarzähne beseitigt und im Falle der Inlays, Onlays und

z.T. Teilkronen wird noch die Restzahnsubstanz berücksichtigt werden die fehlenden Außenflächen ergänzt (Fig. 6 und 8) und wird dann an die Präparationslinie so angepasst, dass ein nahezu glatter harmonischer Übergang erfolgt (Fig. 7). Durch Verschmelzung der Außen- und Innenflächen entlang der Präparationslinie (Randkurve) kann dann das Zahnersatzteil gefräst werden. Entscheidend zum einen ist, dass im Vergleich zu vorher erwähnten bereits bekannten Methoden durch die Auswahl vieler unterschiedlicher Zähne aus einer Zahnbibliothek nicht der Zahn der Situation angepasst wird, sondern ein für die Situation schon sehr gut passender Zahn ausgewählt wird, bei dem dann nur sehr kleine und damit automatisierbare und Fehler unanfalligere Adaptationen durchzuführen sind. Weiterhin werden wichtige oder komplizierte Teile der Zahnoberfläche von weniger wichtigen oder einfacheren Teilen getrennt. Zu ersteren gehört z.B. die Kaufläche, zu zweiten die Vestibulär, Approximal- und Oralflächen der Zähne. Durch diese Aufteilung kann man sich auf die bessere Anpassung der komplizierteren Oberflächen aus der Zahnbibliothek beschränken, während die Außenflächen automatisch ergänzt und rekonstruiert werden. Für die Außenflächen genügt die Angabe nur weniger Konstruktionspunkte (Fig. 8 und 16). Eine Möglichkeit der Implementierung ist die Berechnung von Bezier-, Nurbs- oder B-Spline-Flächen, die stetig und glatt an die entsprechenden Teile der Präparationsgrenze und der Grenze des eingefügten Bibliotheksdatensatzes anschließen und dabei die Konstruktionspunkte (wie z.B. Approximalkontakt, Ausbuchtung der Vestibulär- oder Oralfläche) interpolieren.

Für den Aufbau dieser Zahnbibliothek ist eine Struktur sinnvoll, bei der jedem Zahndatensatz entweder durch Referenzierung oder durch entsprechende Namensgebung ein Datensatz zugeordnet ist, der den Typ und die Merkmale, die für die Auswahl berücksichtigt werden sollen, beinhaltet. Zusätzlich soll die Bibliothek aus Zahnoberflächen bestehen, die von natürlichen kariesfreien und unversehrten Zähnen herrühren.

Die allgemeinste Form der Zahnbibliothek enthält die Gesamtheit aller möglichen vorkommenden natürlicher und auch künstlicher Zahnformen. Sinnvollerweise wird man die Zahnbibliothek in Gruppen mit unterschiedlichen Zahntypen einteilen. Bei dieser Untergruppierung nach dem Zahntyp kann es sich zum Beispiel um Molaren, Prämolaren, Eckzähne und Frontzähne handeln. Als Zahntyp kann aber auch der OK-6er, UK-4er, OK-1er etc. stehen. Möglich ist des weiteren eine Unterscheidung nach Alter und Abrasion, nach Geschlecht, nach Volkszugehörigkeit, nach Größe der Zähne, nach morphologischen Besonderheiten etc, z.B. können die Gruppen OK-7er im Alter von 50-60 Jahren, OK-6er mit und ohne Tuberculum Carabelli, UK-3er bei weiblichen Personen ein Beispiel für einen Zahntyp darstellen. Der Begriff Zahntyp umfasst also eine je nach Auf gabenstellung sehr variable Gruppierungsmöglichkeit.

Bei der Erstellung des generischen Zahnmodelldatensatzes kann man den Faktor Alter bzw. Abrasionsgrad, wobei Zahnbibliotheksflächen eines bestimmten Zahntyps in allen Alters- bzw. Abrasionsstufen vorliegen sollen, berücksichtigen und den oder die ermittelten Kombinationen aus Linearfaktoren und Hauptkomponenten, die diesen Faktor beschreiben, nutzen, um die Abrasion für die jeweilige Restgebisssituation optimal einzustellen.

Es besteht weiterhin die Möglichkeit, bei der Herstellung von Zahnrestaurationen einen Vorschlag für die möglichen Lokalisationen aller Berührungspunkte mit dem Gegenzahn/Gegenbezahnung (d.h. den Berührungsstellen mit dem Gegenkiefer) automatisch zu ermitteln. Dazu wird ein funktionelles Bissregistrat und ein statisches bzw. okklusales Bissregistrat vermessen, die Datensätze im gleichen Koordinatensystem referenziert, so dass es der Situation am Patienten bzw. am Modell entspricht und anschließend alle Bereiche oder Punkte, die einen sehr geringen Abstand von dem einen zum anderen Registrat aufweisen, herausgefiltert. Entscheidend ist, dass außerhalb dieser Bereiche kein Kontaktpunkt liegen kann und darf. Daher könnte sogar die Kontaktpunktgestaltung automatisiert oder zumindest wesentlich vereinfacht werden.

Weiterhin ist es möglich, die Datensätze des Durchschnittszahns, des generischen Zahndatensatzes, der rekonstruierten Zahnersatzteile, der Zahnrestaurationen oder der Zahnmodelle durch Glättung (Filterung) oder spezielle Anpassung an die Werkzeug- bzw. Bearbeitungsgeometrien für den Herstellungsprozess aufzubereiten. Darunter fallen auch Fräserradiuskorrekturen etc.

Alle vorgestellten Verfahren eignen sich für Inlay-, Onlay, Teilkronen-, Kronen- und Brückenversorgungen gleichermaßen. Ausgehend von der rekonstruierten Kaufläche ist auch eine reduzierte Kauflächengestaltung für Gerüste möglich, die gewährleistet, dass die Verblendung nachher immer ungefähr die gleiche Schichtstärke aufweist. Dies kann man erreichen, indem in einem konstanten Abstand von der rekonstruierten Oberfläche die neue Oberfläche berechnet wird oder zumindest durch Abflachung im Bereich der Höcker und Fissuren die Kaufläche dann entsprechend der Schichtstärke in Richtung des präparierten Zahnes verschoben wird.

In Patentanspruch 14 wird die Verwendung einer numerisch gesteuerten Maschine beschrieben, mit deren Hilfe, gesteuert von den ermittelten Datensätzen, die physische Herstellung von Zahnmodellen, Zahnrestaurationen und Zahnersatzteilen erfolgt. Im Prinzip sind alle möglichen automatisierten Fertigungsverfahren wie CNC-Fräsen oder -Schleifen, Laserbearbeitung, Stereolithographie oder lithographische Sinterverfahren einsetzbar. Die Materialpalette für die Zahnrestauration, Zahnersatzteile oder Zahnmodelle kann von Kunststoff über Metalle (Titan, Gold, Stahl etc.) bis zu Keramik reichen. In der Zahnmedizin sind bereits eine Reihe von Materialien speziell für den CAD/CAM-Prozess verfügbar.

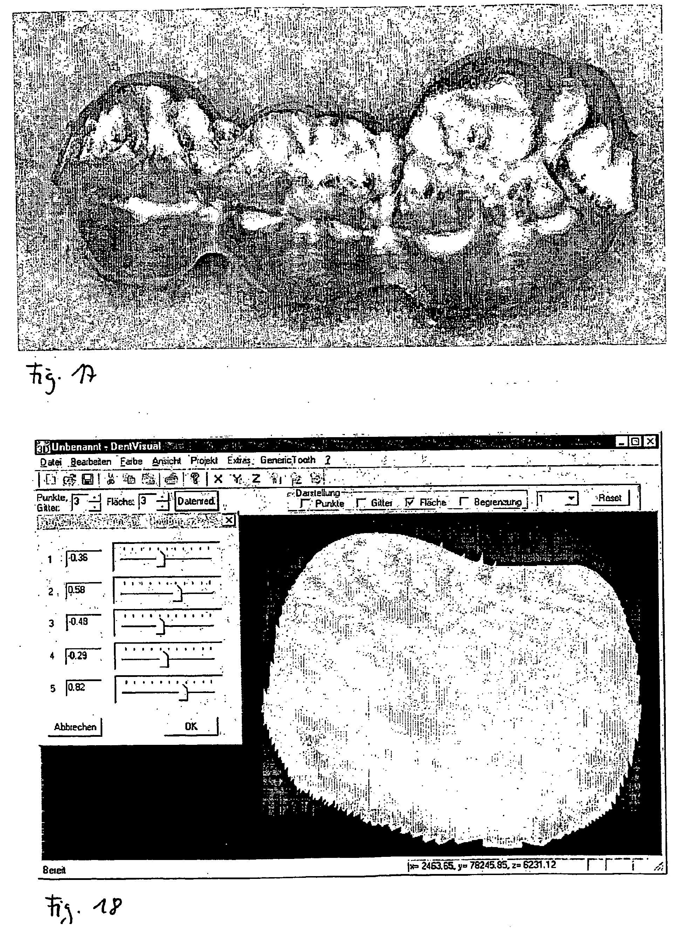

In den Patentansprüchen 15 und 16 werden Vorrichtungen beschrieben, die es ermöglichen, dass für den generischen Zahnmodelldatensatz die Linearfaktoren der zumindest wichtigsten Hauptkomponenten durch eine Regeleinrichtung direkt und interaktiv verändert werden können. Gleichzeitig kann die Auswirkung dieser Veränderung in einer bildlichen Darstellung betrachtet und analysiert werden. In Fig. 18 ist eine Form der Ausgestaltung zu sehen. Die erwähnten Vorrichtungen können z.B. eingesetzt werden, um anstelle der automatischen Rekonstruktion und Optimierung dem Zahnarzt oder Zahntechniker die Möglichkeit zu geben, interaktiv nach eigenen Vorstellungen den generischen Zahnmodelldatensatz an die Restzahnsituation anzupassen.

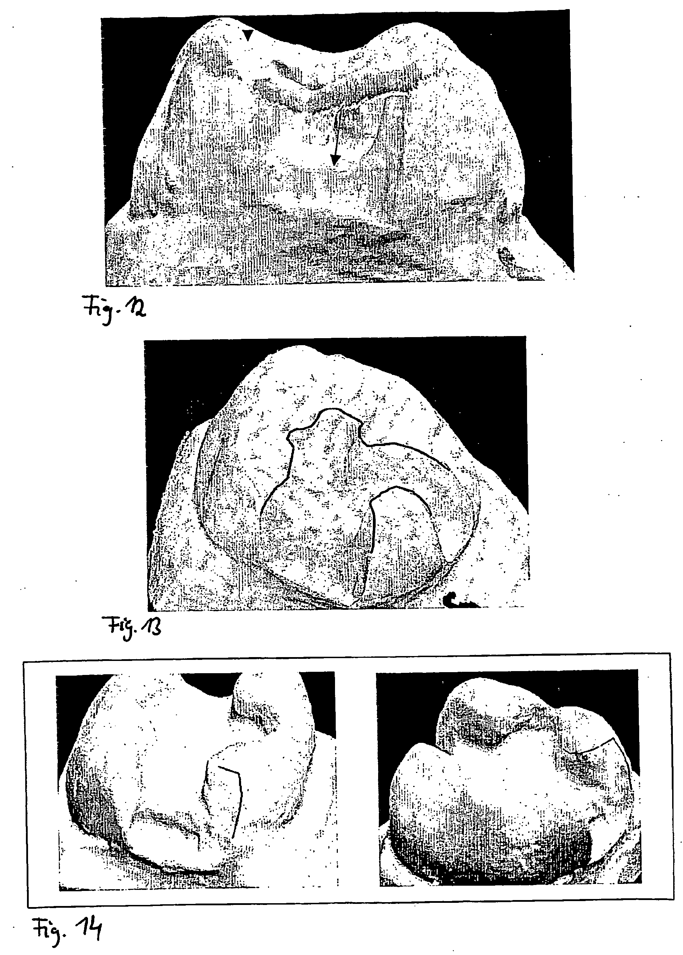

Es ist weiterhin möglich, die gesamten Kauflächenrekonstruktionen durchzuführen, ohne dabei explizit die Restzahnsubstanz ausschneiden oder besonders markieren zu müssen. Vielmehr kann der vollständige Datensatz des reparaturbedürftigen Zahnes herangezogen werden (Fig. 12). Durch Anklicken weniger Startwerte (Korrespondenzpunkte) auf der Restzahnsubstanz wird ein Vorschlag angeboten, anhand dessen für die weitere Iteration oder Anpassungsprozess nur solche Korrespondenzpunkte in Betracht kommen, die unterhalb eines gewissen

Abstandes zwischen vorgeschlagener Zahnfläche und reparaturbedürftigen Zahn liegen (Fig. 12). Die Abstandsschwelle kann auch variiert oder adaptiv angepasst werden. Somit werden mit hoher Wahrscheinlichkeit bei der Rekonstruktion Punkte in der Kavität oder auf den beschliffenen Bereichen der Zahnoberfläche nicht mit berücksichtigt oder fallen durch die geringe Anzahl nicht ins Gewicht. Dadurch wird die automatische Ergänzung der Präparationslinie möglich. Nach erfolgter Rekonstruktion und Anpassung der Kaufläche wird nach den Bereichen gesucht, bei denen ein Übergang von kleineren Abstandswerten (Bereiche, wo sich noch Restzahnsubstanz befindet; hier weist in der Regel die rekonstruierte Kaufläche geringe Abweichungen auf) zu Bereichen mit größeren Abständen (Bereiche wo der Zahn beschliffen oder Zahnsubstanz entfernt wurde) auftritt. In diesen Übergangsbereichen muss auch die Präparationsgrenze oder zumindest Teile davon liegen (Fig. 13). Diese Vorgehensweise kann noch verbessert werden, wenn man in diesen Arealen nach den Stellen der stärksten Krümmung auf der Oberfläche des Datensatzes des reparaturbedürftigen Zahnes sucht und diese Stellen stärkster Krümmung in diesen Bereichen zu einer Linie verbindet (z.B. Fig. 14 und 15). Damit ist von der Rekonstruktion bis zur Präparationsgrenzenfindung ein vollautomatischer Prozess vorstellbar. Man kann dies aber auch als Unterstützung und Vorschlagsunterbreitung für eine weitere interaktive Nacharbeitung seitens des Benutzers einsetzen.

Die Eingabe der Präparationsgrenze ist interaktiv möglich. Hierbei werden in bestimmten Abständen Punkte auf der Oberfläche des elektronischen Bildes des reparaturbedürftigen Zahnes angeklickt. Dieses Anklicken kann mit verschiedenen Steuer- und Kontrollelementen erfolgen, z.B. Computermaus, Tastatur, Joystick oder 3D-Maus. Zwischen diesen ausgewählten Punkten wird jeweils eine Verbindungslinie im Raum interpoliert. Damit man Punkte von der vermessenen Zahnoberfläche bekommt, wird die Verbindungslinie auf die Oberfläche projiziert (Fig. 14). Dabei ist entscheidend, dass die Projektionsrichtung für bestimmte Abschnittsbereiche oder auch für jeden Abschnitt unterschiedlich gewählt werden kann. Dies kann z.B. durch vorprogrammierte Werte erreicht werden oder durch interaktive Einstellung der Ansicht des Zahndatensatzes (Fig. 14). Dies kann z.B. dadurch erfolgen, dass in der Ansicht zur Markierung bzw. Anklicken des jeweiligen Punktes auch die Projektion der Linie in der gleichen Richtung durchgeführt wird. Um einen möglichst

glatten Kurvenverlauf zu bekommen, können die Verbindungslinien neben Geraden auch Spline- oder Parabelsegmente sein. Auch nach erfolgter Projektion auf die Oberfläche können Splinesegmente oder Ähnliches die eventuell zackigen oder verrauschten Kurven noch glätten. Eine besonders sinnvolle Variante besteht noch darin, in der Nähe oder auch zwischen den angeklickten Punkten nach Stellen mit den größten Krümmungen zu suchen. Aufgrund der Vorgehensweise beim Präparieren und Beschleifen eines Zahnes zur Versorgung mit einer Zahnrestauration sind dies die Stellen, wo sich die Präparationsgrenze befinden sollte. Durch Verbinden der Stellen mit den größten Krümmungen, also eine Linie der größten Krümmungen, erhält man schon einen sehr guten Vorschlag für den Verlauf der Präparationsgrenze (Fig. 15).

Eventuell im Datensatz der Zahnrestauration oder Zahnersatzteilen vorkommende Fehlstellen können gefunden und geschlossen werden. Solche Fehlstellen können z.B. dadurch entstehen, dass die rekonstruierte Kaufläche oder der rekonstruierte Datensatz nicht den gesamten beschliffenen Anteil überdeckt oder die Anpassung im Bereich der Präparationsgrenze nicht fehlerfrei erfolgt und daher der Datensatz in diesem Bereich absteht oder Fehler aufweist (Fig. 6, 8, 15). Durch automatischen Vergleich der Präparationslinie mit der Berandungskurve des rekonstruierten Datensatzes kann durch Abstandsprüfung entschieden werden, welche Bereiche der Linien bzw. Kurven zu weit auseinander liegen und daher eine Auffüllung oder Ergänzung notwendig machen (Fig. 15). Nachdem der Startpunkt für Präparationslinie und Berandungskurve nicht identisch sein müssen, müssen die in Frage kommenden Abschnitte der Berandungskurve den entsprechenden Abschnitten der Präparationsgrenze noch automatisch zugeordnet werden. Für die Berechnung der Ergänzungsfläche kann es noch notwendig werden, im Übergangsbereich von dem einen Kurvensegment zu dem anderen Kurvensegment weitere Punkte der jeweiligen Kurven hinzuzufügen, die vorher bei der Abstandsprüfung nicht dem zu ergänzenden Bereich zugeordnet werden konnten und nun ermöglichen, dass eine möglichst geschlossene Linie für die Berechnung der Ergänzungsfläche entsteht. Diese Fehlstellen können geschlossen werden (siehe auch Fig. 16).

Die Kontrolle des Ergebnis oder noch notwendige Interaktionen, die man dem Zahntechniker oder Zahnarzt immer ermöglichen sollte, kann für den Bediener durch Visualisierung mit 3D-Brillen oder 3D-Monitoren etc. erfolgen. Dies ist für den ungeübten Bediener vertrauter.

Die Einbeziehung der Nachbarzähne oder Antagonisten oder der symmetrisch gegenüberliegenden Zahntypen bei der Auswahl der besten Kaufläche ist mittels der generischen Kauflächen und der zugehörigen Hauptkomponenten ebenfalls möglich.

Die Erfindung ist anhand der Ausführungsbeispiele in der Beschreibung und in den Abbildungen lediglich exemplarisch dargestellt und nicht darauf beschränkt, sondern umfasst alle Variationen, Modifikationen, Substitutionen und Kombinationen, die der Fachmann den vorliegenden Unterlagen insbesondere im Rahmen der Ansprüche und der allgemeinen Darstellungen sowie der Beschreibung der Ausführungsbeispiele und deren Darstellungen in den Abbildungen entnehmen und mit seinem fachmännischen Wissen sowie dem Stand der Technik insbesondere unter Einbeziehung der vollständigen Offenbarungsgehalte der in dieser Beschreibung angegebenen älteren Anmeldungen kombinieren kann. Insbesondere sind alle einzelnen Merkmale und Ausgestaltungsmöglichkeiten kombinierbar.

In den Zeichnungen zeigen:

Fig. 1 einen reparaturbedürftigen Zahn;

Fig. 2 ein zu dem reparaturbedürftigen Zahn referenziertes Bissregistrat;

Fig. 3 den Zahn gemäß Fig. 1 dargestellt mit Nachbarzähnen (oben) und zusätzlich mit refernziertem Bissregistrat (unten);

Fig. 4 eine Darstellung des Zahns nach Fig. 1 mit Bissregistrat und ausgewählten Korrespondenzpunkten;

Fig. 5 eine aus einer Zahnbibliothek anhand der Korrespondenzpunkte ausgesuchte Zahnoberfläche;

Fig. 6 rotierte Darstellung der Situation nach Fig. 5 mit erkennbaren Fehlstellen;

Fig. 7 eingepasste und vollständig ergänzte Zahnrestauration;

Fig. 8 eingepasste Zahnfläche für Kronenpräparation mit Einzeichnung von Interpolationspunkten für die Rekonstruktion der noch fehlenden Außenflächen;

Fig. 9 ein Beispiel einer generisch erzeugten Zahnoberfläche mit Korrespondenzpunkten;

Fig. 10 reparaturbedürftiger Zahn mit den der Fig. 9 entsprechenden Korrespondenzpunkten;

Fig. 11 reparaturbedürftiger Zahn mit Bissregistrat und mit den der Fig. 9 entsprechenden Korrespondenzpunkten;

Fig. 12 Beispiel für die Unterscheidung der Bereiche, die zur beschliffenen Zahnsubstanz gehören und durch eine Zahnrestauration aufgefüllt werden müssen, und Bereichen, die zur unversehrten Restzahnsubstanz gehören, anhand der Prüfung des Abstandes während des Rekonstruktions- und Anpassungsprozesses der Außenhülle;

Fig. 13 Beispiel für die Erkennung der Präparationsgrenze am Übergang zwischen den beiden vorher unterschiedenen Bereichen;

Fig. 14 Beispiel für das interaktive Einzeichnen der Präparationsgrenzen unter verschiedenen Ansichten und Projektion der Verbindungslinie auf die Zahnoberflächen;

Fig. 15 Beispiel für das Auffinden noch zu ergänzender Bereiche durch Vergleich der beiden Berandungskurven;

Fig. 16 eine vollständige Zahnrestauration, bei der die noch fehlenden Areale automatisch ergänzt wurden;

Fig. 17 Beispiel für eine nach dem Verfahren des generischen Zahnmodells in einer Maschine angefertigten Zahnrestauration;

Fig. 18 ein Beispiel für eine Regeleinrichtung zur Veränderung der Linearfaktoren und der gleichzeitigen Darstellung der Veränderung;

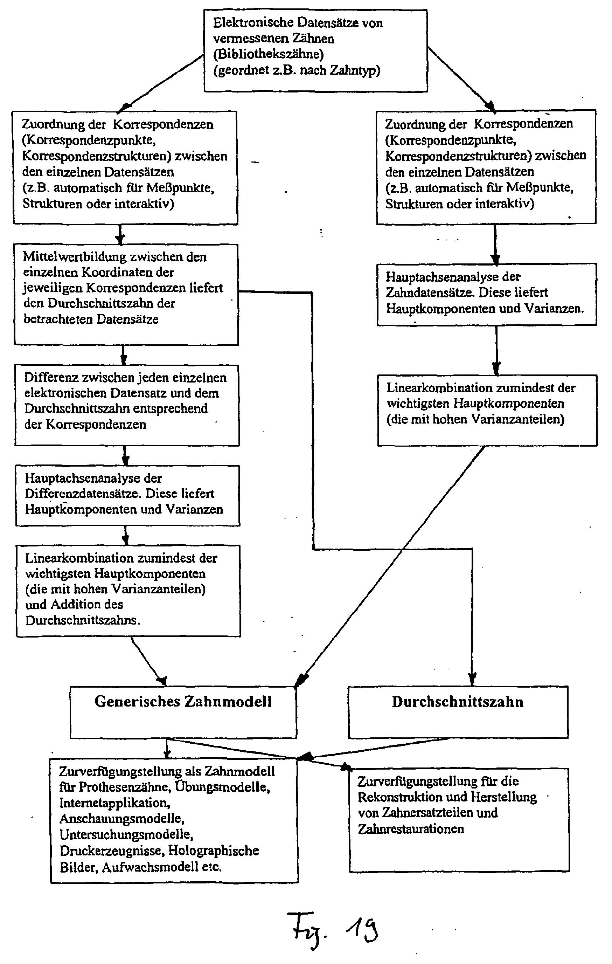

Fig. 19 ein Flussdiagramm für die Erstellung eines Durchschnittsdatensatzes oder eines generischen Zahnmodelldatensatzes;

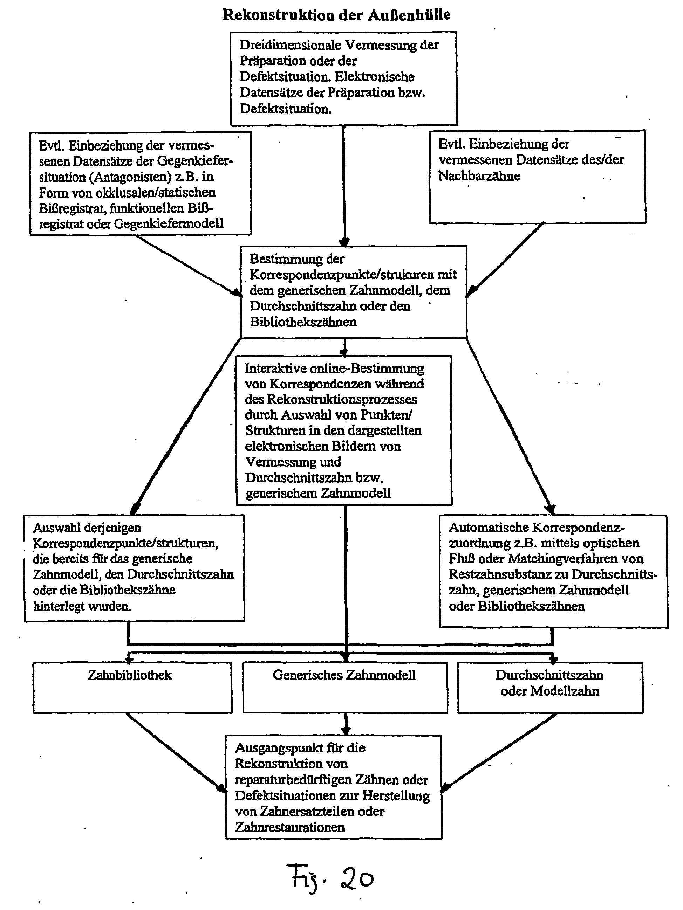

Fig. 20 ein Flussdiagramm für eine Rekonstruktion einer Außenhülle;

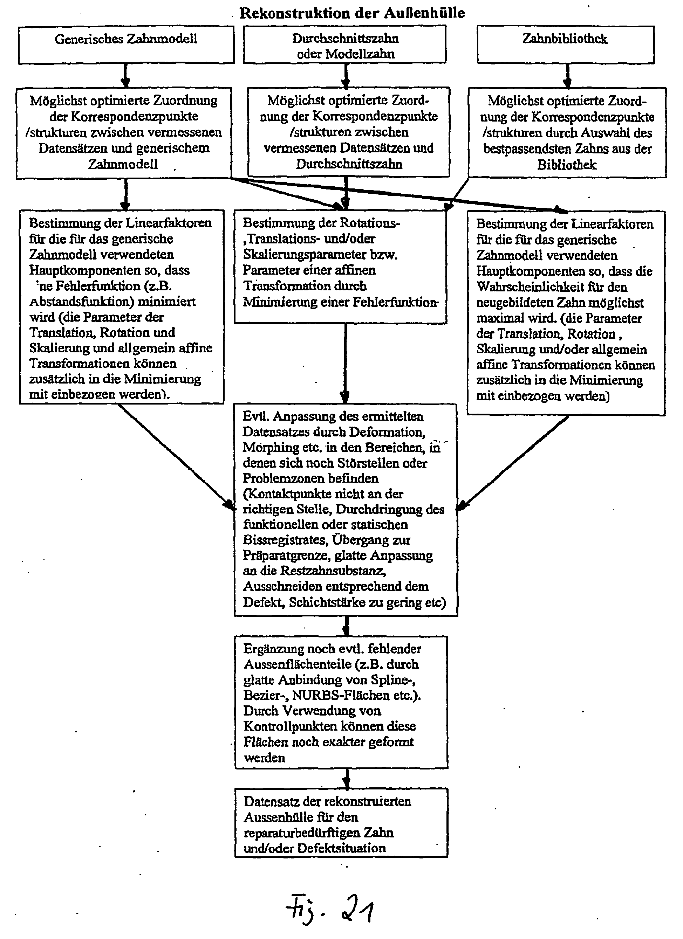

Fig. 21 eine Fortsetzung des Flussdiagramms von Fig. 14 für eine Rekonstruktion einer Außenhülle;

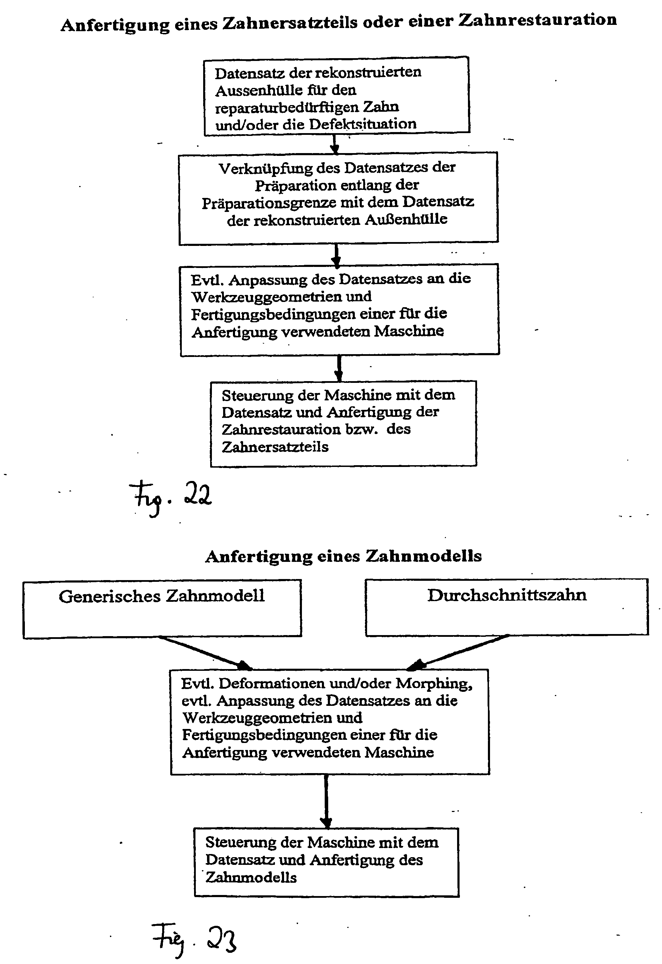

Fig. 22 ein Flussdiagramm für eine Anfertigung eines Zahnersatzteils oder einer Zahnrestauration; und

Fig. 23 ein Flussdiagramm für eine Anfertigung eines Zahnmodells.

Es folgen nun weitere Erläuterungen zur Erfindung und zu Ausführungsformen der Erfindung.

Fig. 1: Zeigt einen dreidimensional vermessenen reparaturbedürftigen Zahn als Höhendatensatz.

Fig. 2: zeigt ein zu einem reparaturbedürftigen Zahn referenziertes Bissregistrat. Dieses Bissregistrat enthält Informationen zum Antagonisten. Es kann sich dabei entweder um ein statisches Bissregistrat und/oder um ein funktionelles Bissregistrat und/oder um die Gegenzahnreihe handeln. Wichtig ist nur, dass diese Informationen ins gleiche Koordinatensystem wie der Zahn referenziert werden.

Fig.3: zeigt die gleiche Situation wie in Fig. 2, jedoch mit Nachbarzähnen (oben) und zusätzlichem Bissregistrat (unten) dargestellt. Die gesamte Anordnung stellt die Restgebisssituation dar. Die Nachbarzähne geben z.B. die Information für die mesial-distale Ausdehnung der rekonstruierten Außenhülle. Außerdem kann anhand der Form der

Nachbarzähne eine Auswahl für die Zahnoberfläche (Außenhülle) getroffen werden, die für die Rekonstruktion in der entsprechenden Situation in Frage kommen.

Gemäß Fig. 4: können durch Markieren von Punkten auf der Restzahnfläche und/oder Kontaktpunkten auf dem Bissregistrat (Gegenzahnreihe) und/oder zum Approximalkontakt des Nachbarzahnes die Zahnoberflächen entweder aus der Bibliothek oder mittels des generischen Zahnes mit Hauptkomponenten optimal durch entsprechende Minimierung einer Fehlerfunktion angepaßt werden. Anstatt der Punktmarkierungen kann man auch größere Bereiche auswählen, wie z.B. Restzahnsubstanz und/oder Kontaktflächen, anhand derer die Zahnoberflächen durch Matching oder optischen Fluß angepaßt werden. Es besteht weiterhin die Möglichkeit, die Lokalisationen möglicher Kontaktpunkte automatisch durch Vergleich des funktionellen Bissregistrates und des statischen (okklusalen) Bissregistrates zu ermitteln.

Fig. 5. zeigt eine Kaufläche aus der Bibliothek ausgesucht und an die Position transformiert oder eine generische Kaufläche, durch Optimierung der Linearfaktoren der Hauptkomponenten an die Situation angepasst. In beiden Fällen erhält man schon ein relativ gutes Resultat, das durch Deformation noch an die Ränder und an die Gegenbezahnung angepasst werden muss.

Gemäß Fig. 6. liefert eine Anpassung von Kauflächen je nach noch vorhandener Restzahnsubstanz fehlende Lücken im Bereich vor allem unterhalb des Zahnäquators. Diese Lücken müssen noch geschlossen werden. Obwohl die Auswahl von vollständigen Zahnoberflächen (d.h. inkl. Außenbereiche) möglich wäre, ist zur Zeit eine getrennte Anpassung von Kaufläche und Außenflächen (Oral-, Vestibulär, Approximalfläche) sinnvoll. Dadurch werden Parameter im Randbereich getrennt von Parametern im Kauflächenbereich behandelt und damit wird in den einzelnen Bereichen eine bessere Anpassung gewährleistet. Außerdem ist der Vorgang der Ergänzung der Kauflächen automatisch durchführbar.

Gemäß Fig. 7 erhält man nach Anpassen an den Rand/ Gegenzahn und Ergänzung der fehlenden Flächen die gesamte Außenkontur (Außenhülle) des Zahnes. Wichtig ist dabei jeweils der glatte Übergang in den Randbereichen. Durch Zusammenfügen dieses Datensatzes an der Präparationsgrenze mit dem Datensatz der vermessenen Kavität/Defekt ist der gewünschte Formkörper für die CNC-Bearbeitung und Anfertigung in einer Produktionsmaschine aufbereitet.

Fig. 8.: Wenn keine oder nur wenig Restzahnsubstanz vorhanden ist (z.B. Kronenpräparationen), erfolgt die Ergänzung der fehlenden Außenflächen über den gesamten zirkulären Bereich. Dabei ist es sinnvoll, einige Konstruktionspunkte vorzugeben. Die Ergänzung wird in der Regel automatisiert ablaufen. Die weitere Anforderung ist ein glatter Übergang in den Randbereichen.

Fig. 9. zeigt ein Beispiel einer generisch erzeugten Zahnoberfläche. Hier handelt es sich z.B. um einen Durchschnittszahn, der aus 200 jugendlichen unversehrten ersten Oberkiefermolaren (OK-6er) berechnet wurde.

Fig. 10. und 11: Die generische Kaufläche mit den Hauptkomponenten kann wiederum an die Restgebisssituation angepasst werden durch Verwendung der Restzahnsubstanz (Fig. 10) und/oder durch Auswahl bestimmter Punkte auf Bissregistrat (Fig.11) und/oder Nachbarzähne etc. Im Gegensatz zur direkten Verwendung einer Zahnbibliothek kann mittels des generischen Zahnmodelldatensatzes die Auswahl von bestimmten Kontakt- und Merkmalspunkten bzw. -strukturen direkt vor der Berechnung und Konstruktion erfolgen, da es genügt, diese Punkte am generischen Zahn zu markieren. In der Zahnbibliothek müsste dagegen jeder einzelne Zahn mit den neuen Merkmalspunkten versehen werden. Dies erlaubt daher auch einen schnellen Wechsel je nach Situation, um verschiedene Okklusions- und Formkonzepte umzusetzen.

**Patentansprüche**

1. Verfahren zur Herstellung eines elektronischen Datensatzes eines für die Anfertigung eines Zahnersatzteils, einer Zahnrestauration oder eines Zahnmodells verwendbaren generischen Zahnmodells, mit folgenden Verfahrensschritten:

   a) durch Vermessen einer vorbestimmten Mindestanzahl von Zähnen gleichen Zahntyps wird eine Vielzahl von elektronischen Datensätzen dieses Zahntyps erzeugt;
   b) es erfolgt eine Zuordnung zumindest einer Anzahl von für diesen Zahntyp charakteristischen Korrespondenzpunkten und/oder Korrespondenzstrukturen in den einzelnen elektronischen Datensätzen;
   c) es wird eine Hauptachsenanalyse für die zugeordneten Korrespondenzpunkte und/oder Korrespondenzstrukturen der vermessenen Zähne durchgeführt;
   d) es wird eine Linearkombination aus mindestens einem Teil der sich ergebenden Hauptachsen für den betrachteten Zahntyp durchgeführt und als generischer Zahnmodelldatensatz zur Verfügung gestellt.

2. Verfahren nach Anspruch 1, bei welchem die Zuordnung der Korrespondenzpunkte und/oder Korrespondenzstrukturen automatisch erfolgt.

3. Verfahren nach einem der Ansprüche 1 oder 2, bei welchem für die Zuordnung der Korrespondenzpunkte und/oder Korrespondenzstrukturen eine gewichtete Kombination zumindest aus Höhenwerten und Steigungen und/oder Krümmungen der entsprechenden elektronischen Daten verwendet wird.

4. Verwendung einer mit dem Verfahren nach einem der Ansprüche 1 bis 3 erhaltenen elektronischen Darstellung eines generischen Zahnmodells als elektronische Vorlage für eine Herstellung von physischen Zahnmodellen, Zahnrestaurationen oder Zahnersatzteilen mittels einer entsprechend dem generischen Zahnmodelldatensatz oder auch mittels Teilen dieser Datensätze gesteuerten Maschine.

5. Verfahren zur Herstellung von physischen Zahnersatzteilen oder Zahnrestaurationen für reparaturbedürftige Zähne oder für Defektsituationen unter Verwendung einer mit dem Verfahren nach einem der Ansprüche 1 bis 4 erhaltenen elektronischen Darstellung eines generischen Zahnmodells, mit folgenden Schritten:

   a) es wird eine dreidimensionale Vermessung einer Präparation des reparaturbedürftigen Zahns oder einer Defektsituation durchgeführt und ein die Präparation oder Defektsituation darstellender elektronischer Datensatz erzeugt;
   b) aus der elektronischen Information der vermessenen Präparation oder der vermessenen Defektsituation werden für den Zahntyp des reparaturbedürftigen Zahns oder für den in die Defektsituation passenden Zahntyp charakteristische Korrespondenzpunkte und/oder Korrespondenzstrukturen ausgewählt;
   c) die Korrespondenzpunkte und/oder Korrespondenzstrukturen im elektronischen Datensatz der vermessenen Präparation oder Defektsituation werden entsprechenden Korrespondenzpunkten und/oder Korrespondenzstrukturen im Datensatz des generischen Zahnmodells zugeordnet;
   d) die einander zugeordneten Korrespondenzpunkte und/oder Korrespondenzstrukturen werden durch ein Optimierungsverfahren möglichst weit gehend angenähert;
   e) der durch die Optimierung ermittelte Datensatz wird einer Rekonstruktion des fehlenden Teils des reparaturbedürftigen Zahns oder zur Ergänzung der Defektsituation zu Grunde gelegt;

f) ein physisches Zahnersatzteil oder eine physische Zahnrestauration für den reparaturbedürftigen Zahn oder fur die Defektsituation wird mittels einer Maschine hergestellt, die entsprechend dem bei dem Schritt e) erhaltenen Datensatz gesteuert wird.

6. Verfahren nach Anspruch 5 zur Herstellung eines dreidimensionalen elektronischen Datensatzes von Zahnersatzteilen oder Zahnrestaurationen, bei welchem nach erfolgter Zuordnung von Korrespondenzpunkten und/oder -Strukturen des reparaturbedürftigen Zahnes und/oder der Defektsituation zum generischen Zahnmodelldatensatz die Linearfaktoren für den verwendeten Teil der Hauptachsen so optimiert werden, dass die neue Linearkombination möglichst gut die Korrespondenzen anpasst oder zur Deckung bringt.

7. Verfahren nach Anspruch 6, bei welchem die Linearfaktoren durch Minimierung der Abstände zwischen den Korrespondenzpunkten ermittelt werden.

8. Verfahren nach Anspruch 6 oder 7, bei welchem die Linearfaktoren so ermittelt werden, dass die Wahrscheinlichkeit für die ermittelte Linearkombination möglichst hoch ist.

9. Verfahren nach einem der Ansprüche 5 bis 8, bei welchem die Optimierung die Wichtigkeit bestimmter Korrespondenzpunkte und/oder Korrespondenzstrukturen in Form von Wichtungsfaktoren berücksichtig.

10. Verfahren nach einem der Ansprüche 5 bis 9, bei welchem elektronische Datensätze eines funktionellen Bissregistrats und/oder eines statischen Bissregistrats berücksichtigt werden.

11. Verfahren nach Anspruch 10, bei welchem die Information des Bissregistrats für die Bildung der Korrespondenzpunkte und/oder Korrespondenzstrukturen zur Rekonstruktion des reparaturbedürftigen Zahns und/oder der Defektsituation einbezogen wird.

12. Verfahren nach Anspruch 10 oder 11, bei welchem die möglichen Bereiche der Kontaktpunkte mit dem/die Gegenzahn/Gegenzähne als Korrespondenzpunkte und/oder Korrespondenzstrukturen ermittelt werden, indem durch Überlagerung des Datensatzes des statischen/okklusalen Bissregistrats mit dem Datensatz des funktionellen Bissregistrats die Bereiche mit geringen Abständen zwischen diesen Bissregistraten ausgewählt werden.

13. Verfahren nach einem der Ansprüche 5 bis 12, bei welchem von mindestens einem Nachbarzahn und/oder mindestens einem Gegenzahn und/oder mindestens einem symmetrisch gegenüberliegenden Zahn abgeleitete elektronische Daten für die Bildung der Korrespondenzpunkte und/oder Korrespondenzstrukturen zur Rekonstruktion des reparahubedürftigen Zahns und/oder der Defektsituation einbezogen werden.

14. Verwendung einer numerisch gesteuerten Maschine zur Herstellung von Zahnmodellen, Zahnrestaurationen oder Zahnersatzteilen, indem die Maschine entsprechend dem mit dem Verfahren nach einem der Ansprüche 1 bis 13 erhaltenen Datensatzes gesteuert wird.

15. Vorrichtung zur Veränderung eines mit einem Verfahren nach einem der Ansprüche 1 bis 3 erhaltenen generischen Zahnmodelldatensatzes, aufweisend eine Regeleinrichtung, mittels welcher die Linearfaktoren zumindest eines Teils der Hauptkomponenten des generischen Zahnmodelldatensatzes veränderbar sind.

16. Vorrichtung nach Anspruch 15, mit einer mit der Regeleinrichtung gekoppelten Darstellungseinrichtung zur bildlichen Darstellung des dem generischen Zahnmodelldatensatzes entsprechenden generischen Zahnes und der Auswirkung eines mittels der Regeleinrichtung durchgeführten Veränderung der Linearfaktoren.

**Claims**

1. A method of creating an electronic data set of a generic tooth model that can be used for creating a dental prosthetic item, a tooth restoration, or a tooth model, having the following process steps:

   a) scanning a predetermined minimum number of teeth of the same tooth type to provide a multiplicity of electronic data sets of this tooth type;
   b) assigning at least a certain number of correspondence points and/or correspondence structures that are characteristic for this tooth type, in the individual electronic data sets;

c) carrying out a principal component analysis for the assigned correspondence points and/or correspondence structures of the scanned teeth;

d) carrying out a linear combination of at least a portion of the resulting principal components for the tooth type of interest and making this available as a generic tooth model data set.

**2.** A method as defined in claim 1, in which the assignment of the correspondence points and/or correspondence structures is carried out automatically.

**3.** A method as defined in any one of claims 1 or 2, in which, for the assignment of the correspondence points and/or correspondence structures, a weighted combination is used taken at least from height values and gradients and/or curvatures of the corresponding electronic data.

**4.** A method of using an electronic representation of a generic tooth model, as obtained using a method as defined in any one of claims 1 through 3, as an electronic template for the creation of physical tooth models, tooth restorations, or dental prosthetic items using a machine that is controlled by the generic tooth model data set, or by parts of these data sets.

**5.** A method of creating physical dental prosthetic items or tooth restorations for defective teeth or for defective dental prosthetic items, using an electronic representation of a generic tooth model, as obtained using a method as defined in any one of claims 1 through 4, comprising the following steps:

a) a three-dimensional scan of a preparation of the defective tooth or of a defective dental prosthetic item is carried out, and an electronic data set is created representing the preparation or defective dental prosthetic item;

b) characteristic correspondence points and/or correspondence structures are selected from the electronic information of the scanned preparation, or of the scanned defective dental prosthetic item, for the tooth type of the defective tooth, or for the tooth type appropriate to the defective dental prosthetic item;

c) the correspondence points and/or the correspondence structures in the electronic data sets of the scanned preparation or defective dental prosthetic item are assigned in accordance with the correspondence points and/or correspondence structures in the data set of the generic tooth model;

d) the correspondence points and/or correspondence structures that are assigned to each other are approximated to the greatest extent possible using an optimization method;

e) the data set obtained by the optimization is made the basis of the reconstruction of the missing part of the defective tooth, or for building up the defective dental prosthetic item;

f) a physical dental prosthetic item or a physical tooth restoration for the defective tooth or for the defective dental prosthetic item is created using a machine that is controlled in accordance with the data set obtained in step e).

**6.** A method as defined in claims 5, for creating a three-dimensional electronic data set of dental prosthetic items or tooth restorations, in which, after the correspondence points and/or structures of the defective tooth and/or the defective dental prosthetic item have been assigned to the generic tooth model data set, the linear factors for the portion of the principal components used are optimized, such that the new linear combination is adapted to the greatest extent possible to the correspondences, or is brought into register therewith.

**7.** A method as defined in claim 6, in which the linear factors are determined by minimizing the distances between the correspondence points.

**8.** A method as defined in claim 6 or 7, in which the linear factors are determined such that the probability for the determined linear combination is as high as possible.

**9.** A method as defined in any one of claims 5 through 8, in which said optimization takes into account the significance of specific correspondence points and/or correspondence structures in the form of weighting factors.

**10.** A method as defined in any one of claims 5 through 9, in which electronic data sets of a functional bite registration and/or a static bite registration are taken into account.

**11.** A method as defined in claim 10, in which the information of the bite registration is taken into account in creating the correspondence points and/or correspondence structures for reconstructing the defective tooth and/or the defective dental prosthetic item.

**12.** A method as defined in claim 10 or claim 11, in which the possible regions of the contact points with the opposing tooth/teeth are determined as correspondence points and/or correspondence structures, in that, by superimposing the data set of the static/occlusal bite registration over the data sets of the functional bite registration, the regions showing short distances between the bite registrations are selected.

**13.** A method as defined in any one of claims 5 through 12, in which, for creating the correspondence points and/or correspondence structures for the reconstruction of the defective tooth and/or the defective dental prosthetic item, electronic data are included that are derived from at least one adjacent tooth and/or at least one opposing tooth and/or at least one symmetrically opposite tooth

**14.** A method of using a numerically controlled machine for creating tooth models, tooth restorations, or tooth dental prosthetic items, **characterized in that** the machine is controlled in accordance with a data set obtained according to a method as defined in any one of claims 1 through 13.

**15.** A device for changing a generic tooth model data set that is obtained as defined in any one of claims 1 through 3, having a control device which can change the linear factors of at least a portion of the principal components of the generic tooth model data set.

**16.** A device as defined in claim 15, having a display device which is coupled to the control device and is adapted to afford a graphic display of the generic tooth corresponding to the generic tooth model data set and to show the effect of a change in the linear factors as carried out by the control device.


**Revendications**

**1.** Procédé de réalisation d'un enregistrement électronique d'un modèle de dent générique utilisable pour la fabrication d'une pièce de prothèse dentaire, d'une restauration dentaire ou d'un modèle de dent, comprenant les étapes de procédé suivantes:

a) en mesurant un nombre minimum prédéterminé de dents du même type de dent, on génère une pluralité d'enregistrements électroniques de ce type de dent;
b) une affectation d'au moins un certain nombre de points de correspondance et/ou de structures de correspondance caractéristiques de ce type de dent est effectuée dans les enregistrements électroniques individuels;
c) une analyse d'axe principal est exécutée pour les points de correspondance et/ou les structures de correspondance affectés des dents mesurées;
d) une combinaison linéaire composée d'au moins d'une partie des axes principaux résultants est exécutée pour le type de dent en question et mise à disposition sous la forme d'un enregistrement de modèle de dent générique.

**2.** Procédé selon la revendication 1, dans lequel l'affectation des points de correspondance et/ou des structures de correspondance est effectuée automatiquement.

**3.** Procédé selon l'une quelconque des revendications 1 ou 2, dans lequel pour l'affectation des points de correspondance et/ou des structures de correspondance, on utilise une combinaison pondérée des données électroniques correspondantes, composée au moins de valeurs de hauteur et de pentes et/ou de courbures.

**4.** Utilisation d'une représentation électronique d'un modèle de dent générique, obtenue par le procédé selon l'une quelconque des revendications 1 à 3, pour une réalisation de modèles de dent, de restaurations dentaires ou de pièces de prothèse dentaire physiques au moyen d'une machine commandée selon l'enregistrement de modèle de dent générique ou bien par des parties de ces enregistrements.

**5.** Procédé de fabrication de pièces de prothèse dentaire ou de restaurations dentaires physiques pour des dents abîmées ou pour des situations d'édentement en utilisant une représentation électronique d'un modèle de dent générique obtenue par le procédé selon l'une quelconque des revendications 1 à 4, comprenant les étapes suivantes:

a) un mesurage en trois dimensions d'une préparation de la dent abîmée ou d'une situation d'édentement est effectué et un enregistrement électronique représentant la préparation ou la situation d'édentement est généré;
b) parmi les informations électroniques de la préparation mesurée ou de la situation d'édentement mesurée, on sélectionne des points de correspondance et/ou des structures de correspondance caractéristiques du type de dent de la dent abîmée ou du type de dent adapté à la situation d'édentement;

c) les points de correspondance et/ou les structures de correspondance dans l'enregistrement électronique de la préparation ou de la situation d'édentement mesurée sont affectés à des points de correspondance et/ou des structures de correspondance adéquats dans l'enregistrement du modèle de dent générique;

d) les points de correspondance et/ou les structures de correspondance affectés les uns aux autres sont dans la mesure du possible approximés par un procédé d'optimisation;

e) l'enregistrement déterminé par l'optimisation est utilisé comme base d'une reconstruction de la partie manquante de la dent abîmée ou pour combler la situation d'édentement;

f) une pièce de prothèse dentaire physique ou une restauration dentaire physique pour la dent abîmée ou pour la situation d'édentement est fabriquée au moyen d'une machine commandée par l'enregistrement obtenu à l'étape e).

6. Procédé selon la revendication 5 pour réaliser un enregistrement électronique tridimensionnel de pièces de prothèse dentaire ou de restaurations dentaires, dans lequel, une fois l'affectation de points et/ou de structures de correspondance de la dent abîmée et/ou de la situation d'édentement à l'enregistrement de modèle de dent générique effectuée, les facteurs linéaires pour la partie utilisée des axes principaux sont optimisés de telle sorte que la nouvelle combinaison linéaire adapte ou fait coïncider les correspondances aussi bien que possible.

7. Procédé selon la revendication 6, dans lequel les facteurs linéaires sont déterminés en minimisant les distances entre les points de correspondance.

8. Procédé selon la revendication 6 ou 7, dans lequel les facteurs linéaires sont déterminés de telle sorte que la probabilité de la combinaison linéaire déterminée est aussi élevée que possible.

9. Procédé selon l'une quelconque des revendications 5 à 8, dans lequel l'optimisation tient compte de la pondération de certains points de correspondance et/ou structures de correspondance sous la forme de facteurs de pondération.

10. Procédé selon l'une quelconque des revendications 5 à 9, dans lequel des enregistrements électroniques d'un enregistrement d'occlusion fonctionnel et/ou d'un enregistrement d'occlusion statique sont pris en compte.

11. Procédé selon la revendication 10, dans lequel les informations de l'enregistrement d'occlusion sont incorporées pour former les points de correspondance et/ou les structures de correspondance en vue de la reconstruction de la dent abîmée et/ou de la situation d'édentement.

12. Procédé selon la revendication 10 ou 11, dans lequel les plages possibles des points de contact avec la ou les dents antagonistes sont déterminées sous la forme de points de correspondance et/ou de structures de correspondance par la sélection des zones à faibles distances entre ces enregistrements d'occlusion en superposant l'enregistrement de l'enregistrement d'occlusion statique/occlusal à l'enregistrement de l'enregistrement d'occlusion fonctionnel.

13. Procédé selon l'une quelconque des revendications 5 à 12, dans lequel des données électroniques dérivées d'au moins une dent voisine et/ou d'au moins une dent antagoniste et/ou d'au moins une dent symétriquement opposée sont incorporées pour former les points de correspondance et/ou les structures de correspondance en vue de la reconstruction de la dent abîmée et/ou de la situation d'édentement.

14. Utilisation d'une machine à commande numérique pour fabriquer des modèles de dent, des restaurations dentaires ou des pièces de prothèse dentaire en commandant la machine selon l'enregistrement obtenu par le procédé selon l'une quelconque des revendications 1 à 13.

15. Dispositif de modification d'un enregistrement de modèle de dent générique obtenu par un procédé selon l'une quelconque des revendications 1 à 3, présentant un dispositif de régulation permettant de modifier les facteurs linéaires au moins d'une partie des principaux composants de l'enregistrement de modèle de dent générique.

16. Dispositif selon la revendication 15, comprenant un dispositif d'imagerie couplé au dispositif de régulation pour l'imagerie de la dent générique correspondant à l'enregistrement de modèle de dent générique et de l'effet d'une modification des facteurs linéaires effectuée au moyen du dispositif de régulation.

Fig. 1

Fig. 2

Fig. 3

Fig. 4

z.B. Korrespondenzpunkte

Fig. 5

Fig. 6

Fig. 7

Fig. 8

Fig. 9

Fig. 10

Fig. 11

z.B. Kontaktpunkte zum Antagonisten

Fig. 12

Fig. 13

Fig. 14

Fig. 15

Fig. 16

Fig. 17

Fig. 18

Elektronische Datensätze von
vermessenen Zähnen
(Bibliothekszähne)
(geordnet z.B. nach Zahntyp)

Zuordnung der Korrespondenzen
(Korrespondenzpunkte,
Korrespondenzstrukturen) zwischen
den einzelnen Datensätzen
(z.B. automatisch für Meßpunkte,
Strukturen oder interaktiv)

Zuordnung der Korrespondenzen
(Korrespondenzpunkte,
Korrespondenzstrukturen) zwischen
den einzelnen Datensätzen
(z.B. automatisch für Meßpunkte,
Strukturen oder interaktiv)

Mittelwertbildung zwischen den
einzelnen Koordinaten der
jeweiligen Korrespondenzen liefert
den Durchschnittszahn der
betrachteten Datensätze

Hauptachsenanalyse der
Zahndatensätze. Diese liefert
Hauptkomponenten und Varianzen.

Differenz zwischen jeden einzelnen
elektronischen Datensatz und dem
Durchschnittszahn entsprechend
der Korrespondenzen

Linearkombination zumindest der
wichtigsten Hauptkomponenten
(die mit hohen Varianzanteilen)

Hauptachsenanalyse der
Differenzdatensätze. Diese liefert
Hauptkomponenten und Varianzen

Linearkombination zumindest der
wichtigsten Hauptkomponenten
(die mit hohen Varianzanteilen)
und Addition des
Durchschnittszahns.

**Generisches Zahnmodell**

**Durchschnittszahn**

Zurverfügungstellung als Zahnmodell
für Prothesenzähne, Übungsmodelle,
Internetapplikation,
Anschauungsmodelle,
Untersuchungsmodelle,
Druckerzeugnisse, Holographische
Bilder, Aufwachsmodell etc.

Zurverfügungstellung für die
Rekonstruktion und Herstellung
von Zahnersatzteilen und
Zahnrestaurationen

Fig. 19

## Rekonstruktion der Außenhülle

Dreidimensionale Vermessung der Präparation oder der Defektsituation. Elektronische Datensätze der Präparation bzw. Defektsituation.

Evtl. Einbeziehung der vermessenen Datensätze der Gegenkiefersituation (Antagonisten) z.B. in Form von okklusalen/statischen Bißregistrat, funktionellen Bißregistrat oder Gegenkiefermodell

Evtl. Einbeziehung der vermessenen Datensätze des/der Nachbarzähne

Bestimmung der Korrespondenzpunkte/strukuren mit dem generischen Zahnmodell, dem Durchschnittszahn oder den Bibliothekszähnen

Interaktive online-Bestimmung von Korrespondenzen während des Rekonstruktionsprozesses durch Auswahl von Punkten/ Strukturen in den dargestellten elektronischen Bildern von Vermessung und Durchschnittszahn bzw. generischem Zahnmodell

Auswahl derjenigen Korrespondenzpunkte/strukturen, die bereits für das generische Zahnmodell, den Durchschnittszahn oder die Bibliothekszähne hinterlegt wurden.

Automatische Korrespondenz-zuordnung z.B. mittels optischen Fluß oder Matchingverfahren von Restzahnsubstanz zu Durchschnittszahn, generischem Zahnmodell oder Bibliothekszähnen

Zahnbibliothek

Generisches Zahnmodell

Durchschnittszahn oder Modellzahn

Ausgangspunkt für die Rekonstruktion von reparaturbedürftigen Zähnen oder Defektsituationen zur Herstellung von Zahnersatzteilen oder Zahnrestaurationen

Fig. 20

## Rekonstruktion der Außenhülle

| Generisches Zahnmodell | Durchschnittszahn oder Modellzahn | Zahnbibliothek |
|---|---|---|

| Möglichst optimierte Zuordnung der Korrespondenzpunkte /strukturen zwischen vermessenen Datensätzen und generischem Zahnmodell | Möglichst optimierte Zuordnung der Korrespondenzpunkte /strukturen zwischen vermessenen Datensätzen und Durchschnittszahn | Möglichst optimierte Zuordnung der Korrespondenzpunkte /strukturen durch Auswahl des bestpassendsten Zahns aus der Bibliothek |
|---|---|---|

| Bestimmung der Linearfaktoren für die für das generische Zahnmodell verwendeten Hauptkomponenten so, dass ˙ne Fehlerfunktion (z.B. Abstandsfunktion) minimiert wird (die Parameter der Translation, Rotation und Skalierung und allgemein affine Transformationen können zusätzlich in die Minimierung mit einbezogen werden). | Bestimmung der Rotations- ,Translations- und/oder Skalierungsparameter bzw. Parameter einer affinen Transformation durch Minimierung einer Fehlerfunktion | Bestimmung der Linearfaktoren für die für das generische Zahnmodell verwendeten Hauptkomponenten so, dass die Wahrscheinlichkeit für den neugebildeten Zahn möglichst maximal wird. (die Parameter der Translation, Rotation , Skalierung und/oder allgemein affine Transformationen können zusätzlich in die Minimierung mit einbezogen werden) |
|---|---|---|

Evtl. Anpassung des ermittelten Datensatzes durch Deformation, Morphing etc. in den Bereichen, in denen sich noch Störstellen oder Problemzonen befinden (Kontaktpunkte nicht an der richtigen Stelle, Durchdringung des funktionellen oder statischen Bissregistrates, Übergang zur Präparatgrenze, glatte Anpassung an die Restzahnsubstanz, Ausschneiden entsprechend dem Defekt, Schichtstärke zu gering etc)

Ergänzung noch evtl. fehlender Aussenflächenteile (z.B. durch glatte Anbindung von Spline-, Bezier-, NURBS-Flächen etc.). Durch Verwendung von Kontrollpunkten können diese Flächen noch exakter geformt werden

Datensatz der rekonstruierten Aussenhülle für den reparaturbedürftigen Zahn und/oder Defektsituation

Fig. 21

## Anfertigung eines Zahnersatzteils oder einer Zahnrestauration

Datensatz der rekonstruierten
Aussenhülle für den
reparaturbedürftigen Zahn
und/oder die Defektsituation

Verknüpfung des Datensatzes der
Präparation entlang der
Präparationsgrenze mit dem Datensatz
der rekonstruierten Außenhülle

Evtl. Anpassung des Datensatzes an die
Werkzeuggeometrien und
Fertigungsbedingungen einer für die
Anfertigung verwendeten Maschine

Steuerung der Maschine mit dem
Datensatz und Anfertigung der
Zahnrestauration bzw. des
Zahnersatzteils

Fig. 22

## Anfertigung eines Zahnmodells

Generisches Zahnmodell

Durchschnittszahn

Evtl. Deformationen und/oder Morphing,
evtl. Anpassung des Datensatzes an die
Werkzeuggeometrien und
Fertigungsbedingungen einer für die
Anfertigung verwendeten Maschine

Steuerung der Maschine mit dem
Datensatz und Anfertigung des
Zahnmodells

Fig. 23

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 5217375 A
- EP 0643948 A
- EP 0634150 A
- EP 0913130 A2
- WO 0239056 A
- EP 0643948 A1
- DE 19838239 A1
- DE 19923978 A1
- US 5257203 A
- DE 19642247

### In der Beschreibung aufgeführte Nicht-Patentliteratur

- **MATTIOLA, A. ; MÖRMANN, W.H. ; LUTZ, F.** Computerunterstützte Okklusion von Cerec 2 Inlays und Overlays. *Schweiz Monatsschr Zahnmed,* 1995, vol. 105, 1283-1290
- **KUNZELMANN, K.-H. ; MEHL, A. ; PELKA, M.** Automatische Rekonstruktion von Kauflächen computergenerierter Restaurationen. *Zahnärztl Welt/Rundschau,* 1993, vol. 102, 695-703
- **MEHL, A. ; GLOGER, W. ; HICKEL, R.** Erzeugung von CAD-Datensätzen für Inlays und Kronen mit funktionellen Kauflächen. *Dtsch Zahnärztl Z,* 1997, vol. 52, 520-524
- Designing a CEREC crown. **SALIGER, G.** Cerec 10 year anniversary Smposium. Quintessence, 1996
- **SHELTON, C.R.** 3D Correspondence. *Master Thesis, Massachusetts Institute of Technologie,* 1998
- **UMEYAMA S.** Least-squares estimation of transformation parameters between two point patterns. *IEEE PAMI,* 1991, vol. 13 (4), 276-280
- **BLANZ, V. ; ROMDHANI, S.** Face Identification across different poses and illuminations with a 3D morphable model. *Proc. Int. Conference on Automatic Face and Gesture Recognition,* 2002, 202-207